(19) Europäisches Patentamt
European Patent Office
Office européen des brevets

(11) Publication number: **0 166 609 A2**

# (12) EUROPEAN PATENT APPLICATION

(21) Application number: **85304548.2**

(22) Date of filing: **26.06.85**

(51) Int. Cl.[4]: **C 07 D 487/04**, A 61 K 31/495, A 61 K 31/505 // (C07D487/04, 241:00, 235:00), (C07D487/04, 239:00, 235:00)

(30) Priority: **27.06.84 GB 8416295**
**27.06.84 GB 8416296**
**27.06.84 GB 8416297**
**08.03.85 GB 8506043**
**08.03.85 GB 8506044**
**08.03.85 GB 8506045**

(43) Date of publication of application: **02.01.86 Bulletin 86/1**

(84) Designated Contracting States: **AT BE CH DE FR GB IT LI LU NL SE**

(71) Applicant: **THE WELLCOME FOUNDATION LIMITED, 183-193 Euston Road, London NW1 2BP (GB)**

(72) Inventor: **Barraclough, Paul, Langley Court, Beckenham Kent (GB)**
Inventor: **Smith, Steven, Langley Court, Beckenham Kent (GB)**
Inventor: **Iyer, Ramachandran, Langley Court, Beckenham Kent (GB)**
Inventor: **Nobbs, Malcolm Stuart, Langley Court, Beckenham Kent (GB)**

(74) Representative: **Garrett, Michael et al, The Wellcome Foundation Limited Group Patents and Agreements Langley Court, Beckenham Kent BR3 3BS (GB)**

## (54) Aryl derivatives of heterobicyclic compounds.

(57) The invention relates to use of compounds of formula (I)

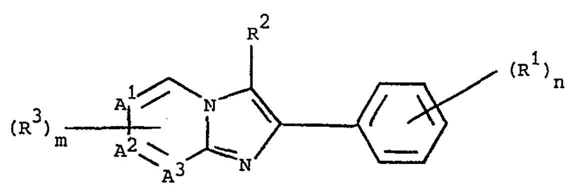

(I)

wherein
n is 1, 2 or 3;
m is 0, 1, 2 or 3;
any one of $A^1$, $A^2$ and $A^3$ is nitrogen and the other two are CH;
$R^1$ groups are independently selected from those specified in (a) and (b) below, namely:
(a) cyano, hydroxy, groups of formula $-S(O)_xR^a$ in which x is 0, 1 or 2 and $R^a$ is a $C_{1-4}$ alkyl group and groups of formula $-OR^b$ in which $R^b$ is allyl or a $C_{1-4}$ alkyl group, which alkyl group is optionally substituted by one or more radicals selected from halo, $C_{1-4}$ alkoxy and groups of formula $-S(O)_xR^a$ in which x and $R^a$ are as defined above;
(b) $C_{1-4}$ alkanoyl, $C_{1-4}$ alkanoylamino, 2-methyl-1,3-dioxalan-2-yl, sulphamoyl, N-$C_{1-4}$ alkylsulphamoyl, N, N-di-$C_{1-4}$ alkylsulphamoyl, $C_{1-4}$ alkylsulphonyloxy, $C_{1-4}$ alkylsulphonylamino, aminosulphonyloxy, N-$C_{1-4}$ alkylaminosulphonyloxy, N,N-di-$C_{1-4}$ alkylaminosulphonyloxy, ureido, 3-$C_{1-4}$ alkylureido, 3,3-di-$C_{1-4}$ alkylureido, aminosulphonylamino, ($C_{1-4}$ alkylaminosulphonyl)amino, (di-$C_{1-4}$ alkylaminosulphonyl)-animo, carboxy, carboxylic derivatives (selected from carbamoyl, N-$C_{1-4}$ alkylcarbamoyl, N, N-di-$C_{1-4}$ alkylcarbamoyl, carboxylic acid halides, $C_{1-4}$ alkoxycarbonyl, hydroxy-$C_{1-4}$ alkoxycarbonyl and hydroxymethylene) and groups of formula $-OR^c$ in which $R^c$ is a straight or branched aliphatic moiety having 1 to 4 carbon atoms, optionally substituted by one or more radicals independently selected from halo, $C_{1-4}$ alkoxy and groups of formula $-S(O)_xR^a$ in which x and $R^a$ are as defined above, provided that $R^c$ is not a group as defined for $R^b$ above;
$R^2$ is selected from hydrogen, halo, amino, hydroxy, $C_{1-4}$ alkyl and $C_{1-4}$ alkoxy;
$R^3$ groups are independently selected from halo, carboxy, amino, $C_{1-4}$ alkylamino hydroxy, $C_{1-4}$ alkoxy, hydroxy-$C_{1-4}$ alkoxy, $C_{1-4}$ alkylthio and $C_{1-4}$ alkylsulphonyloxy;
provided that when $R^2$ is hydrogen and either m is 0 or m is 1 and $R^3$ represents halo, then at least one $R^1$ group must be selected from (b) above;
and physiologically acceptable salts and/or N-oxides thereof, as inotropic agents, and to pharmaceutical formulations containing them.

EP 0 166 609 A2

The invention also includes compounds of formula (I) (including physiologically acceptable salts and/or their N-oxides) *per se*, with the proviso that when n and m are both 1, and $R^2$ is H, then when $A^1$ is nitrogen, $A^2$ and $A^3$ are both CH and $R^1$ is a 4-methoxy group, $R^3$ is not a 7-methoxy group, and when $A^3$ is nitrogen and $A^1$ and $A^2$ are both CH and $R^1$ is a 4-methylsulphonyl group, $R^3$ is not a 6-methoxy group, and processes for their preparation.

A709

0166609

# ARYL DERIVATIVES OF HETEROBICYCLIC COMPOUNDS

The present invention relates to novel imidazobicyclic compounds, processes for their preparation, pharmaceutical formulations containing the compounds and their use in medicine.

Various classes of substituted phenyl-imidazobicyclic compounds have been found previously to be positive inotropic agents, i.e. they are capable of stimulating heart muscle contractions. In particular, UK Patent Specification No. 2 132 203 A describes certain substituted-phenyl imidazopyrimidines, -pyrazines and -triazines alleged to be inotropic agents having minimal effects on blood pressure and heart rate. We have now found that the group of compounds of formula (I) defined hereinbelow, are surprisingly advantageous inotropic agents by virtue of their primary inotropic (cardiotonic) effect and/or secondary pharmacological actions. The formula (I) is defined thus:-

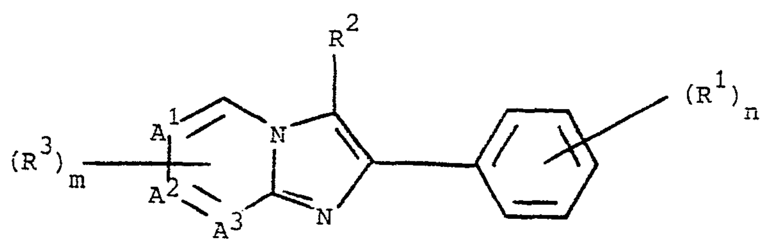

(I)

wherein

n is 1, 2 or 3;

m is 0, 1, 2 or 3;

any one of $A^1$, $A^2$ and $A^3$ is nitrogen and the other two are CH;

$R^1$ groups are independently selected from those specified in (a) and (b) below, namely:-

(a) cyano, hydroxy, groups of formula $-S(O)_xR^a$ in which x is 0, 1 or 2 and $R^a$ is a $C_{1-4}$ alkyl group and groups of formula $-OR^b$ in which $R^b$ is allyl or a $C_{1-4}$ alkyl group, which alkyl group is optionally substituted by one or more radicals selected from halo, $C_{1-4}$ alkoxy and groups of formula $-S(O)_xR^a$ in which x and $R^a$ are as defined above;

(b) $C_{1-4}$ alkanoyl, $C_{1-4}$ alkanoylamino, 2-methyl-1,3-dioxalan-2-yl, sulphamoyl, N-$C_{1-4}$ alkylsulphamoyl, N,N-di-$C_{1-4}$alkylsulphamoyl, $C_{1-4}$ alkylsulphonyloxy, $C_{1-4}$ alkylsulphonylamino, aminosulphonyloxy, N-$C_{1-4}$alkylaminosulphonyloxy, N,N-di-$C_{1-4}$ alkylaminosulphonyloxy, ureido, 3-$C_{1-4}$ alkylureido, 3,3-di-$C_{1-4}$ alkylureido, aminosulphonylamino, ($C_{1-4}$alkylaminosulphonyl)amino,(di-$C_{1-4}$ alkylaminosulphonyl) amino, carboxy, carboxlic derivatives (selected from carbamoyl, N-$C_{1-4}$ alkylcarbamoyl, N,N-di-$C_{1-4}$ alkylcarbamoyl, carboxylic acid halides, $C_{1-4}$ alkoxycarbonyl, hydroxy-$C_{1-4}$alkoxycarbonyl and hydroxymethylene) and groups of formula $-OR^c$ in which $R^c$ is a straight or branched aliphatic moiety having 1 to 4 carbon atoms, optionally substituted by onr or more radicals independently selected from halo, $C_{1-4}$ alkoxy and groups of formula $-S(O)_xR^a$ in which x and $R^a$ are as defined above, provided that $R^c$ is not a group as defined for $R^b$ above;

$R^2$ is selected from hydrogen, halo, amino, hydroxy, $C_{1-4}$ alkyl and $C_{1-4}$ alkoxy;

$R^3$ groups are independently selected from halo, carboxy, amino, $C_{1-4}$alkylamino hydroxy, $C_{1-4}$ alkoxy, hydroxy-$C_{1-4}$alkoxy, $C_{1-4}$ alkylthio and $C_{1-4}$alkylsulphonyloxy;

provided that when $R^2$ is hydrogen and either m is 0 or m is 1 and $R^3$ represents halo, then at least one $R^1$ group must be selected from (b) above.

Unless the context requires otherwise, any reference herein to a compound of formula (I) is to be taken also as a reference to the N-oxides of such a compound, as well as to salts of said compound and the salts of said N-oxides. In particular, where $R^1$ does not represent one or more carboxy or carboxylic acid derivative groups, such salts may be acid addition salts. These salts may be formed by protonation of one or more of the basic ring nitrogen atoms in formula (I) and when intended for therapeutic use, are those salts which are physiologically acceptable, for example, those derived from hydrochloric, hydrobromic, phosphoric, malic, maleic, fumaric, citric, sulphuric, lactic or tartaric acids.

The present invention provides the compounds of formula (I) and their physiologically acceptable salts and N-oxides for use as inotropic agents, especially for the treatment or prevention of congestive heart failure or myocardial insufficiency. The invention also extends to the use of the compounds in the manufacture of therapeutic agents for the treatment of these conditions.

The compounds of formula (I) and their physiologically acceptable salts and N-oxides are useful as inotropic/cardiotonic agents since they have been found in both in vitro and in vivo experiments to produce a positive inotropic effect at low concentrations. The particular advantages of the present compounds in comparison with the known inotropic agents described in the aforementioned UK Patent Specification, as demonstrated by the results of tests described hereinafter, include one or more of high inotropic potency, a mitigation or lack of vasodilation and a prolonged duration of action.

Most of the compounds of formula (I) (including the physiologically acceptable salts and N-oxides thereof) are believed to be novel, and these constitute a primary feature of the present invention. The novel compounds are those of formula (I) (as hereinbefore defined) with the proviso that:-

when n and m are both 1, and $R^2$ is H, then when $A^1$ is nitrogen, $A^2$ and $A^3$ are both CH and $R^1$ is a 4-methoxy group, $R^3$ is not a 7-methoxy group, and when $A^3$ is nitrogen and $A^1$ and $A^2$ are both CH and $R^1$ is a 4-methylsulphonyl group, $R^3$ is not a 6-methoxy group.

The compound of formula (I) wherein $A^3$ is nitrogen and $A^1$ and $A^2$ are both CH, n and m are both 1, $R^1$ is a 4-methylsulphonyl group, $R^2$ is H and $R^3$ is a 6-methoxy group, is described in L. Almirante et al, J.Med.Chem., 9, 29-33(1966). The compound is reported therein as having analgesic and anti-inflammatory activities in the rat. The compound wherein $A^1$ is nitrogen and $A^2$ and $A^3$ are both CH, n and m are both 1, $R^1$ is a 4-methoxy group, $R^2$ is H and $R^3$ is a 7-methoxy group is described in Rogul'chenko et al, Farm. Zh. (Kiev) 1976, (4), 29-32 but is ascribed no medical therapeutic properties.

In the case where $R^1$ represents one or more groups selected from carboxy and carboxylic acid derivatives, the salts may be those derived from bases. For therapeutic use, such base salts are those which are physiologically acceptable, for example ammonium salts, alkali metal salts such as sodium and potassium salts, alkaline earth metal salts such as calcium and magnesium salts, and salts with organic bases such as dicyclohexylamine salts.

As well as physiologically acceptable salts (whether derived from acids or bases) the present invention also includes other salts which may be used for purifying or characterising the parent compounds of formula (I) and their N-oxides.

Among the various aspects of the present invention which hereinafter may be claimed are any of the subclasses of the compounds of formula (I), i.e. subclasses consisting of any combinations of groups specified for the substituents $R^1$-$R^3$, any of the permitted variants $A^1$-$A^3$ for the imidazobicyclic ring system and any of the permitted values for n and m.

The subclasses which may be claimed include those defined in the following list which is not intended to be exhaustive or limiting:-

(i) compounds wherein $A^1$ is N and $A^2$ and $A^3$ are both CH;
(ii) compounds wherein $A^2$ is N and $A^1$ and $A^3$ are both CH;
(ii) compounds wherein $A^3$ is N and $A^1$ and $A^2$ are both CH;
(iv) compounds wherein $R^1$ groups are selected only from (a);
(v) compounds wherein $R^1$ groups are selected only from (b);
(vi) compounds wherein at least one $R^1$ group is carbamoyl;
(vii) compounds wherein at least one $R^1$ group is other than carbamoyl;
(viii) compounds wherein $R^2$ is hydrogen;
(ix) compounds wherein m is 0;
(x) compounds wherein at least one $R^3$ group is methoxy;
(xi) any combination of two or more of the groups of compounds specified in (i)- (xi) above.

One group of compounds of formula (I) comprises those wherein:-

n is 2;

m is 0 or 1;

$R^1$ groups are independently selected from cyano, hydroxy, groups of formula -$S(O)_xR^a$ in which and $R^a$ are as defined in formula (I), groups of formula -$OR^b$ in which $R^b$ is a $C_{1-4}$ alkyl group, $C_{1-4}$akanoyl, $C_{1-4}$ alkanoylamino, sulphamoyl, N-$C_{1-4}$ alkylsulphamoyl, N,N,-di-$C_{1-4}$alkylsulphamoyl, $C_{1-4}$ alkylsulphonyloxy, $C_{1-4}$alkylsulphonylamino, ureido and carbamoyl (and optionally also aminosulphonylamino); and

$R^2$ is selected from hydrogen, halo, amino and hydroxy;

$R^3$ (where present) is selected from halo, amino, $C_{1-4}$alkylamino, hydroxy, $C_{1-4}$alkoxy, hydroxy-$C_{1-4}$alkoxy, $C_{1-4}$alkythio and $C_{1-4}$alkylsulphonyloxy.

Another group of compounds of formula (I) comprises those wherein $R^1$ groups are independently selected from:-

cyano, hydroxy, groups of formula $-S(O)_xR^a$ in which x is 0, 1 or 2 and $R^a$ is a $C_{1-4}$ alkyl group, sulphamoyl, N-$C_{1-4}$ alkylsulphamoyl, N,N-di-$C_{1-4}$alkylsulphamoyl, $C_{1-4}$ alkylsulphonyloxy, $C_{1-4}$ alkylsulphonylamino, aminosulphonyloxy, N-$C_{1-4}$ alkylaminosulphonyloxy,N-N-di-$C_{1-4}$ alkylaminosulphonyloxy, carboxy, carboxylic acid derivatives (selected from carbamoyl, N-$C_{1-4}$alkylcarbamoyl, N,N-di-$C_{1-4}$ alkylcarbamoyl, carboxylic acid halides, $C_{1-4}$ alkoxycarbonyl, hydroxy-alkoxycarbonyl and hydroxymethylene) and groups of formula $-OR^d$ in which $R^d$ is a straight or branched aliphatic moiety having 1 to 4 carbon atoms, optionally substituted by one or more radicals independently selected from halo, $C_{1-4}$ alkoxy and groups of formula $-S(O)_xR^a$ in which x and $R^a$ are as defined above;

$R^2$ is selected from hydrogen, hydroxy, $C_{1-4}$ alkyl and $C_{1-4}$ alkoxy;

$R^3$ groups are independently selected from halo, carboxy, amino, hydroxy, $C_{1-4}$ alkoxy, $C_{1-4}$ alkylthio and $C_{1-4}$alkylsulphonyloxy; with the further provisos that:-

when $A^2$ is nitrogen and $A^1$ and $A^3$ are both CH, then $R^3$ is other than amino or $C_{1-4}$ alkylsulphonyloxy, and when $A^2$ is nitrogen and $A^1$ and $A^3$ are both CH, or $A^3$ is nitrogen and $A^1$ and $A^2$ are both CH, then $R^3$ is other than hydroxy.

In general formula (I), alkyl groups (whether alone or as part of alkyl containing groups such as alkoxy) may be straight or branched and selected from methoxy, ethoxy, propoxy and butoxy groups.

In the definition of $R^1$, the aliphatic group represented by $R^c$ may for example be a $C_{1-4}$ alkenyl (other than alkyl) or $C_{1-4}$ alkynyl group. In particular, $R^1$ may for example represent one or more groups selected from cyano, $C_{1-4}$ alkanoyl, carboxy, carbamoyl, methylthio, methylsulphinyl, methylsulphonyl, methoxy, ethoxy, n-propoxy, iso-propoxy, propargyloxy, sulphamoyl, N-$C_{1-4}$ alkylsulphamoyl, N,N-di-$C_{1-4}$ alkylsulphamoyl, $C_{1-4}$alkylsulphonyloxy, $C_{1-4}$ alkylsulphonylamino, aminosulphonyloxy, N-$C_{1-4}$ alkylaminosulphonyloxy and N,N-di-$C_{1-4}$

alkylaminosulphonyloxy. In the definitions of $R^b$, $R^c$ and $R^3$, halo may be independently fluoro, chloro, bromo or iodo.

Some preferred compounds of formula (I) are those wherein n is 1, 2 or 3 (most preferably 2) m is 0, or 1 (most preferably 0), $R^1$ represents a substituent in the 4-position of the phenyl ring selected from cyano, $C_{1-4}$ alkanoyl (especially acetyl), carboxy or a carboxylic acid derivative (especially carbamoyl) group, a sulphamoyl, N-$C_{1-4}$ alkylsulphamoyl, N,N-di-$C_{1-4}$ alkylsulphamoyl, $C_{1-4}$alkylsulphonyloxy, $C_{1-4}$ alkylsulphonylamino, aminosulphonyloxy, N-$C_{1-4}$alkylaminosulphonyloxy, or N,N-di-$C_{1-4}$ alkylaminosulphonyloxy group (especially when the alkyl group(s) in any of the preceding represent methyl) and groups of formula -$S(O)_xR^a$ as defined (especially when x is 1 and $R^a$ is a methyl group); and/or one, two or three (as appropriate) $C_{1-4}$ alkoxy (especially methoxy) group(s) in the 2- or 3-(and where permissible, 4-position(s) of the phenyl ring, $R^2$ is hydrogen and $R^3$ (when present) is a halo or $C_{1-4}$ alkoxy group in the 6- or the 8-position halo or of the imidazopyrazine ring system.

A preferred compound by virtue of its advantageous pharmacological properties is 2-(2-methoxy-4-carbamoylphenyl)-imidazo[1,2-a]pyrazine and its N-oxides and physiologically acceptable salts.

Other preferred compounds include the following, their N-oxides and physiologically acceptable salts:-

8-methoxy-2-(2-methoxy-4-methylsulphinylphenyl) imidazo [1,2-a]pyrazine
2-(4-methylsulphonyloxy-2-methoxyphenyl)-8-methoxy-imidazo[1,2-a]pyrazine
2-(4-carbamoyl-2-methoxyphenyl)-8-methoxy-imidazo[1,2-a]pyrazine

Other examples of compounds of formula (I) and their acid addition salts include the following bases and their N-oxides and physiologically acceptable salts:-

2-(2-methoxy-4-methylsulphonyloxyphenyl)imidazo[1,2-a]pyrazine
8-methoxy-2-(2-methoxy-4-methylthiophenyl)imidazo[1,2-a]pyrazine
8-methoxy-2-(2-methoxy-4-methylsulphonyphenyl)imidazo[1,2-a]pyrazine
8-chloro-2-(2-methoxy-4-acetylphenyl)imidazo[1,2-a]pyrazine
2-(2-methoxy-4-carboxyphenyl)-imidazo[1,2-a]pyrazine
2-(3-methoxy-4-carbamoylphenyl)-imidazo[1,2-a]pyrazine
2-(3-methoxy-4-carboxyphenyl)-imidazo[1,2-a]pyrazine
2-(2-methoxy-4-sulphamoylphenyl)imidazo[1,2-a]pyrazine
2-(2-methoxy-4-(N-methylsulphamoyl)phenyl)imidazo[1,2-a]pyrazine

2-(2-methoxy-4-(N,N-dimethylsulphamoyl)phenyl)imidazo[1,2-a]pyrazine
2-(2-methoxy-4-methoxycarbonylphenyl)imidazo[1,2-a]pyrazine
2-(2-methoxy-4-methylsulphonylaminophenyl)imidazo[1,2-a]pyrazine
8-methoxy-2-(2-methoxy-4-methylsulphonylaminophenyl)imidazo[1,2-a]pyrazine
2-(2-methoxy-4-(N-methylaminosulphonyloxy)phenyl)imidazo[1,2-a]pyrazine
2-(2-methoxy-4-acetylphenyl)imidazo[1,2-a]pyrazine
2-(2-methoxy-4-acetylphenyl)-8-methoxy-imidazo[1,2-a]pyrazine
2-(4-hydroxy-2-methoxyphenyl)-8-methoxyimidazo[1,2-a]pyrazine
8-methylamino-2-(2-methoxy-4-methylsulphinylphenyl)imidazo[1,2-a]pyrazine
8-(2-hydroxyethoxy)-2-(2-methoxy-4-methylsulphinylphenyl)imidazo[1,2-a]pyrazine
8-n-propoxy-2-(2-methoxy-4-methylsulphinylphenyl)imidazo[1,2-a]pyrazine
8-methoxy-2-(2-methoxy-4-cyanophenyl)imidazo[1,2-a]pyrazine
8-hydroxy-2-(2-methoxy-4-methylsulphinylphenyl)imidazo[1,2-a]pyrazine
6-methoxy-2-(2-methoxy-4-methylsulphinylphenyl)imidazo[1,2-a]pyrazine
8-methylthio-2-(2-methoxy-4-methylsulphinylphenyl)imidazo[1,2-a]pyrazine
3-bromo-2-(2-methoxy-4-methylsulphinylphenyl)imidazo[1,2-a]pyrazine
2-(2-methoxy-4-[2-methyl-1,3-dioxalan-2-yl]phenyl)imidazo[1,2-a]pyrazine
8-methoxy-2-(2-methoxy-4-[2-methyl-1,3-dioxalan-2-yl]phenyl)imidazo[1,2-a]pyrazine
2-(2-methoxy-4-methylsulphinylphenyl)-7-methoxyimidazo[1,2-a]pyrimidine
3-amino-2-(2,4-dimethoxyphenyl)imidazo[1,2-a]pyrimidine
6-amino-2-(2,4-dimethoxyphenyl)imidazo[1,2-a]pyrimidine
2-(2,4-dimethoxyphenyl)-3-hydroxyimidazo[1,2-a]pyrimidine
6-methoxy-2-(2,4-dimethoxyphenyl)imidazo[1,2-a]pyrimidine
2-(2-methoxy-4-methylsulphonyloxyphenyl)imidazo[1,2-a]pyrimidine
2-(2-methoxy-4-propargyloxyphenyl)imidazo[1,2-a]pyrimidine
6-methoxy-2-(2-methoxy-4-methysulphinylphenyl)-imidazo[1,2-a]pyrimidine
6-amino-2-(2-methoxy-4-methylsulphinylphenyl)-imidazo[1,2-a]pyrimidine
6-hydroxy-2-(2-methoxy-4-methylsulphinylphenyl)-imidazo[1,2-a]pyrimidine
6-methylsulphonyloxy-2-(2-methoxy-4-methylsulphinyphenyl)-imidazo[1,2-a]pyrimidine
6-carboxy-2-(2-methoxy-4-methylsulphinylphenyl)-imidazo[1,2-a]pyrimidine
2-(2-methoxy-4-carboxyphenyl)-imidazo [1,2-a]pyrimidine
2-(2-methoxy-4-carbamoylphenyl)-imidazo[1,2-a]pyrimidine
2-(3-methoxy-4-carbamoylphenyl)-imidazo[1,2-a]pyrimidine
2-(3-methoxy-4-carboxyphenyl)-imidazo[1,2-a]pyrimidine
2-(2-methoxy-4-sulphamoylphenyl)-imidazo[1,2-a]pyrimidine
2-(2-methoxy-4-(N-methylsulphamoyl)phenyl)imidazo[1,2-a]pyrimidine
2-(2-methoxy-4-(N,N-dimethylsuphamoyl)phenyl)imidazo[1,2-a]pyrimidine
2-(2-methoxy-4-methoxycarbonylphenyl)-imidazo[1,2-a]pyrimidine
2-(2-methoxy-4-methylsulphonylaminophenyl)imidazo[1,2-a]pyrimidine

2-(2-methoxy-4-(N-methylaminosulphonyloxy)phenyl)imidazo[1,2-a]pyrimidine
2-(2-methoxy-4-acetylphenyl)imidazo[1,2-a]pyrimidine
2-(2-methoxy-4-methylsulphonyloxyphenyl)-6-methoxy-imidazo[1,2-a]pyrimidine
2-(2-methoxy-4-carbamoylphenyl)-6-methoxy-imidazo[1,2-a]pyrimidine
2-(2-methoxy-4-acetylphenyl)-6-methoxy-imidazo[1,2-a]pyrimidine
2-(2-methoxy-4-methylsulphonyloxyphenyl)imidazo[1,2-c]pyrimidine
2-(2-methoxy-4-propargyloxyphenyl)-imidazo[1,2-c]pyrimidine
7-methoxy-2-(2-methoxy-4-methysulphinylphenyl)-imidazo[1,2-c]pyrimidine
7-amino-2-(2-methoxy-4-methylsulphinylphenyl)-imidazo[1,2-c]pyrimidine
2-(2-methoxy-4-carboxyphenyl)-imidazo[1,2-c]pyrimidine
2-(2-methoxy-4-carbamoylphenyl)-imidazo[1,2-c]pyrimidine
2-(3-methoxy-4-carbamoylphenyl)-imidazo[1,2-c]pyrimidine
2-(3-methoxy-4-carboxyphenyl)-imidazo[1,2-c]pyrimidine
2-(2-methoxy-4-sulphamoylphenyl)-imidazo[1,2-c]pyrimidine
2-(2-methoxy-4-(N-methylsulphamoyl)phenyl)imidazo[1,2-c]pyrimidine
2-(2-methoxy-4-(N,N-dimethylsulphamoyl)phenyl)imidazo[1,2-c]pyrimidine
2-(2-methoxy-4-methoxycarbonylphenyl)-imidazo[1,2-c]pyrimidine
2-(2-methoxy-4-methylsulphonylaminophenyl)imidazo[1,2-c]pyrimidine
2-(2-methoxy-4-(N-methylaminosulphonyloxy)phenyl)imidazo[1,2-c]pyrimidine
2-(2-methoxy-4-acetylphenyl)imidazo[1,2-c]pyrimidine
2-(2-methoxy-4-methylsulphonyloxyphenyl)-7-methoxy-imidazo[1,2-c]pyrimidine
2-(2-methoxy-4-carbamoylphenyl)-7-methoxy-imidazo[1,2-c]pyrimidine
2-(2-methoxy-4-acetylphenyl)-7-methoxy-imidazo[1,2-c]pyrimidine
2-(4-acetamido-2-methoxyphenyl)-8-methoxyimidazo[1,2-a]pyrazine
2-(2-methoxy-4-sulphamoylphenyl)-8-methoxyimidazo[1,2-a]pyrazine
2-(2-methoxy-4-N-methylsulphamoylphenyl)-8-methoxyimidazo[1,2-a]pyrazine
2-(2-methoxy-4-N,N-dimethylsulphamoylphenyl)-8-methoxyimidazo[1,2-a]pyrazine
2-(4-acetamido-2-methoxyphenyl)imidazo[1,2-a]pyrazine
2-(2-methoxy-4-ureidophenyl)imidazo[1,2-a]pyrazine
2-(2-methoxy-4-ureidophenyl)-8-methoxyimidazo[1,2-a]pyrazine
2-(2-methoxy-4-ureidophenyl)imidazo[1,2-a]pyrimidine
2-(2-methoxy-4-aminosulphonylaminophenyl)imidazo[1,2-a]pyrazine
2-(2-methoxy-4-aminosulphonylaminophenyl)-8-methoxyimidazo[1,2-a]pyrazine

The compounds of formula (I) and their salts and N-oxides may be administered by the oral, rectal or parenteral route. In general, these compounds may be administered at a dosage in the range of 1 to 1200 mg per day although the precise dosage will naturally depend on a number of clinical factors, for example, the type (i.e. human or animal), age and weight of the subject, the condition under treatment and its

severity, and the particular compound employed. For administration of the compounds by the oral route, a dosage regime of 100 to 400 mg, e.g. about 200 mg per day may be used, while for administration by the parenteral route, especially intravenously, a dosage regime of 20 to 300 mg, advantageously 35 to 70 mg, e.g. about 50 mg per day is generally preferred. The compounds may be administered intravenously by infusion, if desired, in which case, a dosage rate of, for example, 1-4 mg/min may be employed.

The compounds of formula (I) and their physiologically acceptable acid addition salts and N-oxides of such compounds and salts are preferably administered in the form of pharmaceutical formulations.

The present invention thus further provides pharmaceutically acceptable formulations comprising at least one compound of formula (I) (as defined above) or a physiologically acceptable acid addition salt or an N-oxide of the said compound or salt, in association with at least one pharmaceutical carrier or excipient. The pharmaceutical formulations may be adapted for oral, parenteral (particularly intravenous) or rectal administration. The formulations may conveniently be presented in unit dosage form and may be prepared by any of the methods well known in the art of pharmacy. Such methods include the step of bringing into association the active ingredient with the carrier which may comprise one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable

machine a mixture of the powdered compound moistened with an inert liquid diluent. The tablets may optionally be coated or scored and may be formulated so as to provide slow or controlled release of the active ingredient therein.
Formulations for rectal administration may be presented as a suppository with the usual carriers such as cocoa butter.

Formulations suitable for parenteral administration include aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials,and may be stored in a freeze-dried (lyophilized) condition requiring only the addition of the sterile liquid carrier, for example water for injections, immediately prior to use. Extemporaneous injection solutions and suspensions may be prepared from sterile powder, granules and tablets of the kind previously described.

Preferred unit dosage formulations are those containing a daily dose or unit daily sub-dose, as hereinabove recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavouring agents.

The compounds of formula (I) and their acid addition salts and N-oxides may be prepared by any convenient process apparent to those skilled in the art.

Thus, for example, according to a further feature of the present invention, we provide a process for the preparation of compounds of formula (I) and their N-oxides and acid addition salts which comprises
a) reacting a compound of formula

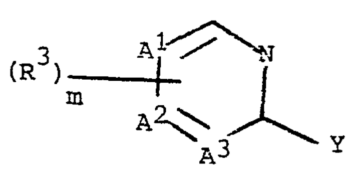

(II)

(wherein $A^1$, $A^2$, $A^3$, m and $R^3$ are as defined above and Y is an amino group or a displaceable radical, e.g. hydrogen or a halogen atom such as chlorine) with a compound of formula

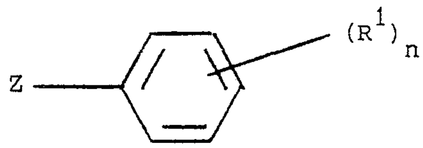

(III)

(wherein $R^1$ and n are as defined above or precursor groups therefor and Z represents a group capable of reacting with group Y to form an imidazo ring system with consequential formation of a compound of formula (I) or an acid addition salt thereof);

b) reacting a compound of formula

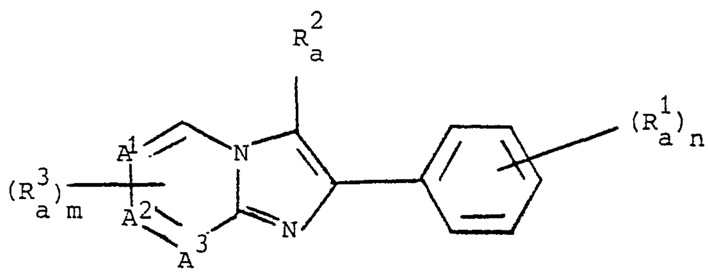

(IV)

(wherein $A^1$, $A^2$, $A^3$, n and m are as defined above and each of $R^1_a$ to $R^3_a$ independently represents a group as defined respectively for $R^1$ to $R^3$ above or a precursor group therefor, provided that at least one of $R^1_a$ to $R^3_a$ represents such a precursor group) or an acid addition salt thereof with an agent serving to effect conversion of the said precursor group(s) into the desired group(s);

c) radical arylation of a compound of formula

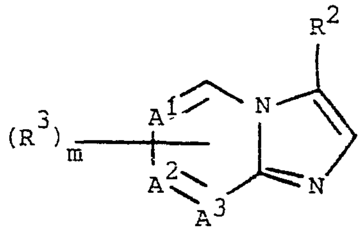

(V)

(wherein $A^1$, $A^2$, $A^3$, $R^2$, $R^3$, and m are as hereinbefore defined) by reaction with a compound of formula

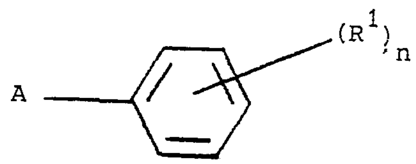

(VI)

(wherein $R^1$ and n are as hereinbefore defined and A represents a displaceable radical, e.g. a diazonium halide, for example the chloride); or

(d) reacting a compound of formula

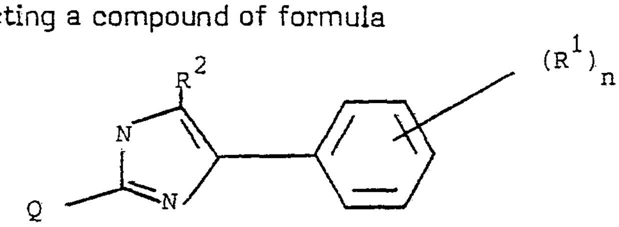

(VII)

(wherein $R^1$, $R^2$ and n are as defined above) with a reagent such that the reagent and Q (in formula (VII)) are chosen so as to react to complete formation of the ring

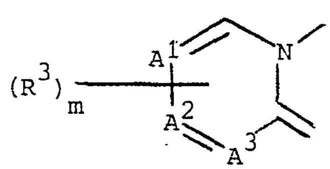

(wherein $A^1$, $A^2$, $A^3$, $R^3$ and m are as defined above);

and where a compound of formula (I) is formed, optionally converting the said compound into an N-oxide or acid addition salt thereof.

When appropriate, processes according to the present invention may involve the steps of protection and subsequent de-protection, such as are well known in chemistry.

The reaction in process a) may optionally be carried out in a solvent, e.g. an organic solvent, for example ethanol, conveniently at an elevated temperature, e.g. up to the reflux temperature of the reaction mixture.

According to a preferred embodiment of process a) above according to the invention, a compound of formula (II) (wherein $R^3$ and m are as hereinbefore defined and Y represents an amino group) is reacted with a compound of formula (III) (wherein $R^1$ and n are as hereinbefore defined and Z is a group of formula $XCH_2CO$- wherein X represents a displaceable radical, preferably halo, i.e. fluoro, chloro, bromo or iodo).

In process a), where the Z group in the compound of formula (III) is a group of formula $XCH_2CO$-, then it may react sequentially with Y and the nitrogen atom in the 1-position of the pyrazine ring in the compound of formula (II), e.g. with the initial formation of a compound of formula.

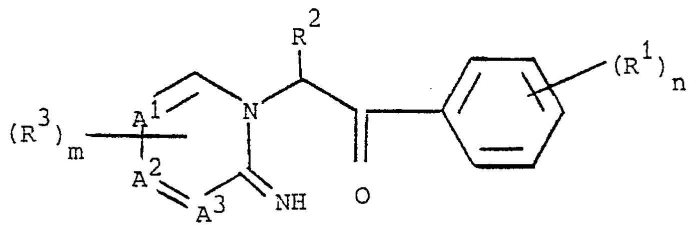

(VIII)

or a compound of formula

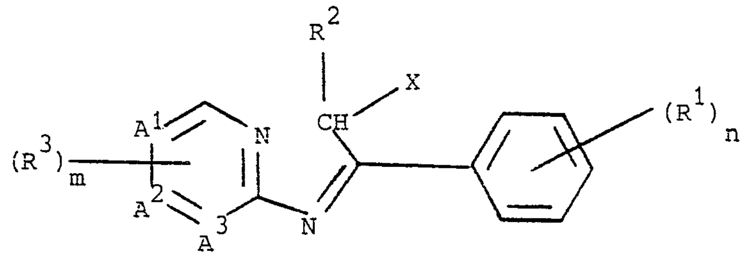

(IX)

(wherein $A^1$ to $A^3$, $R^1$ to $R^3$, X, m and n are as hereinbefore defined) which may subsequently be cyclised, eg. by heating to form a compound of formula (I).

With regard to process b) above, compounds of formula (I) wherein any $R^3$ group represents a $C_{1-4}$alkoxy group may for example be prepared by alkoxylation of a corresponding compound of formula (IV) wherein corresponding $R_a^3$ group(s) represent chlorine.

Compounds of formula (I) wherein any $R^1$ group represents a $C_{1-4}$alkylsulphinyl or -sulphonyl group may be prepared for example by oxidation, e.g. using hydrogen peroxide, organic peracids, or bromine or one of its addition compounds, eg. an alkali metal hypobromite (as described in German Offenlegungsschrift DE 3044 497) of the corresponding $C_{1-4}$alkylthio compound.

Compounds wherein any $R^1$ group represents a carbamoyl group may be prepared by treatment of a corresponding compound of formula (IV) wherein corresponding $R^1_a$ group(s) represent 2-methyl -1,3-dioxalan-2-yl, with a strong acid such as 2N hydrochloric acid, or by subjecting a corresponding compound of formula (IV) wherein corresonding $R^1_a$ group(s) represent cyano, to acid hydrolysis under appropriate conditions, eg using a strong mineral acid such as sulphuric, at room temperature.

Compounds wherein any $R^1$ group represents an alkoxy group may be prepared by treatment of a corresponding compound of formula (IV) wherein corresponding $R^1_a$ group(s) represent hydroxy, with a alkylating agent, e.g. an alkyl sulphate.

Compounds wherein any $R^1$ group represents sulphamoyl, N-$C_{1-4}$alkylsulphamoyl or N,N-di-$C_{1-4}$ alkylsulphamoyl may be prepared by reaction of the corresponding compound of formula (IV) wherein corresponding $R^1_a$ group(s) represent a halo- (eg chloro-) sulphonyl group, with a compound of formula $Q^1Q^2NH$ wherein $Q^1$ and $Q^2$ are independently hydrogen or $C_{1-4}$ alkyl as appropriate.

Compounds wherein any $R^1$ group represents $C_{1-4}$ alkanoylamino may be prepared by reaction of the corresponding compound of formula (IV) wherein corresponding $R^1_a$ group(s) represent amino, with the appropriate acid anhydride, preferably in a suitable medium such as pyridine.

Compounds wherein any $R^1$ group represents ureido may be prepared by reaction of the corresponding compound of formula (IV) wherein corresponding $R^1_a$ group(s) represent amino, with cyanic acid, preferably formed *in situ*, eg from an alkali metal cyanate such as potasium cyanate and a dilute mineral acid such as hydrochloric.

Compounds wherein any $R^1$ represents a $C_{1-4}$ alkylthio group may be prepared by diazotisation of a corresponding compound of formula (IV) wherein $R_a^1$ represents an amino group (e.g. by treatment with nitrous acid), followed by reaction of the product with an appropriate $C_{1-4}$ alkyl mercaptan.

The radical arylation in process c) above may be conveniently effected by a process analogous to the general method of M-H Hung and L.M. Stock, J. Org. Chem., 1982, 47, 448-453. When the compound of formula (VI) is employed in the form of a diazonium salt, this salt may be prepared in conventional manner e.g. from the corresponding amino compound.

In process (d), for the preparation of compounds of formula (I) wherein $A^1$ is nitrogen and $A^2$ and $A^3$ are both CH, the substituent Q in the compound of formula (VII) may be 2-aminoethyl and the reagent then may have formula

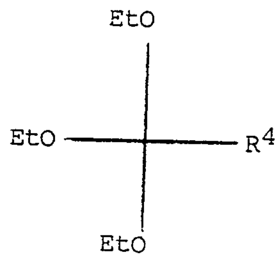

(X)

(wherein $R^4$ is hydrogen or a group as defined for $R^3$ above).

For the preparation of compounds of formula (I) wherein $A^2$ is nitrogen and $A^1$ and $A^3$ are both CH, the substituent Q in the compound of formula (VII) may be formyl and the reagent then may have the formula

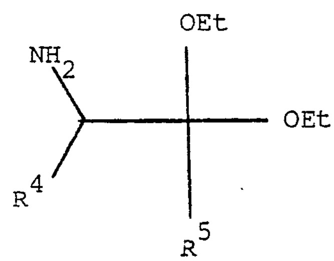

(XI)

(wherein $R^4$ and $R^5$ are independently selected from hydrogen and groups as defined for $R^3$ above).

For the preparation of compounds of formula (I) wherein $A^3$ is nitrogen and $A^1$ and $A^2$ are both CH, the substituent Q in the compound of formula (VII) may be amino and the reagent then may have the formula

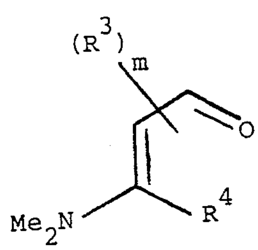

(XII)

(wherein m is 0, 1 or 2 and $R^3$ groups are as defined for $R^3$ above and $R^4$ is hydrogen or a group as defined for $R^3$ above).

The acid addition salts of the compounds of formula (I) may be prepared in conventional manner, e.g. by treatment of the free base with an appropriate acid. The N-oxides of the compounds of formula (I) may also be prepared in conventional manner e.g. by oxidation of the parent compound with an appropriate oxidising agent, eg. m-chloroperbenzoic acid.

Compounds of formula (III) wherein Z is a group of formula $XCH_2CO$- may be prepared conveniently by reaction of a compound of formula (XIII)

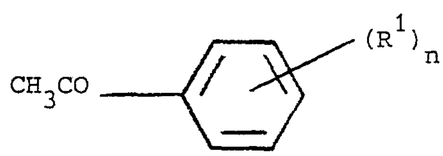

(XIII)

(wherein $R^1$ and n are as defined above) with a halogenating agent such as a copper II halide, eg. the bromide, or suphuryl chloride and where any $R^1$ group(s) represent precursor group(s), converting said precursor group(s) to the desired group(s).

Compounds of formula (III) may also be prepared by reaction of the appropriate acid chloride with diazomethane to yield the corresponding diazoketone, followed by treatment with an acid of formula HX (where X represents halo).

Compounds of (XIII) may be prepared by reaction of the appropriate acid with methyl-lithium, or the appropriate acid chloride with a malonate.

The following Examples illustrate the present invention.

Example 1

2-(2-Methoxy-4-carbamoylphenyl)-imidazo[1,2-a]pyrazine and its hydrochloride

(i) 2-Methoxy-4-cyanobenzoylchloride

2-Methoxy-4-cyanobenzoic acid (5.31g, 30m.mol) was suspended in dry toluene (50ml) and thionylchloride (8ml) was added and the mixture stirred and heated at reflux for 2 hrs. Solvent was removed *in vacuo* and excess thionylchloride was azeotroped with toluene.

(ii) 2-Methoxy-4-cyanoacetophenone

Bis (trimethylsilyl) malonate (15g, 60 mmol) was added to dry ether (150 ml) and the solution stirred under $N_2$ and cooled to - 60°C. n-Butyl lithium (37.5 ml of 1.6M solution in hexane, 60 mmol) was added during 15 min resulting in the formation of a white precipitate of the lithium salt. This was allowed to warm to 0°C and then a solution of 2-methoxy-4-cyanobenzoylchloride (30 mmol) in tetrahydrofuran (15 ml) was added dropwise over 10 minutes. Stirring was continued at 0-5°C for 30 minutes and at room temperature for 18 hours.

The resulting mixture was shaken with saturated $NaHCO_3$, the organic phase dried over $MgSO_4$ and evaporated. The aqueous phase was acidifed to pH 2 with 2N HCl and extracted twice with ethyl acetate These extracts were also dried and evaporated and the two residues combined and dissolved in dioxan (80ml). The solution was heated at reflux for 2 1/2hrs., solvent was removed *in vacuo* and the residue dissolved in dichloromethane and washed with saturated $NaHCO_3$ solution and with water. The organic layer was dried ($MgSO_4$) and evaporated *in vacuo* to give the title product m.pt 111°C.

(iii) 2'-Bromo-2-methoxy-4-cyanoacetophenone

Copper (II) bromide (19.6g, 90 mmol) was suspended in ethyl acetate (150ml) and stirred at room temperature under nitrogen. A solution of 2-methoxy-4-cyano acetophenone (7.68g, 40 mmol) in dry $CHCl_3$ (150 ml) was added dropwise over 1 1/2 hr. At the end of addition the reaction mixture was refluxed for 3 1/2 hrs. The mixture was decolourised with animal charcoal, filtered and evaporated *in vacuo* to give a pale green solid which was triturated with ether to give an off white solid. m.pt. 117-120°C.

(iv) 2-(2-Methoxy-4-cyanophenyl)imidazo-[1,2-a]pyrazine

2-methoxy-4-cyano-2'-bromoacetophenone (20g) and amino pyrazine (7.41g) were dissolved in DMF (100ml) and the mixture heated at $80^o$ under nitrogen atmosphere for 2 hrs. The reaction mixture was poured into water and the insoluble dark brown solid was filtered and dried. The pH of the filtrate was adjusted to 8.0 with 0.88 ammonia. The solid that precipitated was filtered and dried. T.L.C. indicated the two solids to be identical and so they were combined, dissolved in hot chloroform and decolourised with animal charcoal. On evaporation *in vacuo* a pale brown solid was obtained as product. m.p. 199-201°C.

0166609

(v) 2-(2-Methoxy-4-carbamoylphenyl)-imidazo-[1,2-a]pyrazine

2-(2-methoxy-4-cyanophenyl)imidazo-[1,2-a]pyrazine (0.5g) was dissolved in Conc. $H_2SO_4$ (10ml) and the mixture stood at R.T. for 24 hrs. The mixture was poured onto crushed ice (100g). The pH of the resulting solution was carefully adjusted (with cooling) to 7.4 with 10N NaOH. The precipitated solid was filtered, washed with water and dried. m.p. 262-265°C.

(vi) 2-(2-Methoxy-4-carbamoylphenyl)-imidazo-[1,2-a]pyrazine hydrochloride

2-(2-methoxy-4-carbamoylphenyl)imidazo- [1,2-a]pyrazine (0.4g) was suspended in methanol and dry hydrogen chloride gas was passed through the suspension for a few minutes. The mixture was cooled and the solid filtered and dried, m.p. 266-268°C.

Example 2

8-Methoxy-2-(2-methoxy-4-methylthiophenyl)imidazo[1,2-a]pyrazine

(i) 8-Chloro-2-(2-methoxy-4-methylthiophenyl)imidazo[1,2-a]pyrazine

The title compound was prepared in a manner analogous to that described in Example 1 but using 2-amino-3-chloropyrazine in place of aminopyrazine.

(ii) 8-Methoxy-2-(2-methoxy-4-methylthiophenyl)imidazo[1,2-a] pyrazine

The title compound was prepared by treatment of the compound of (i) with sodium methoxide in methanol, m.pt. 132-135 °C.

Example 3

8-methoxy-2-(2-methoxy-4-methylsulphinylphenyl)imidazo[1,2-a]pyrazine

The title compound was made by oxidation of the compound of Example 2 with MCPBA (1 eq), m.p. 184-185°C.

Example 4

8-methoxy-2-(2-methoxy-4-methylsulphonyloxyphenyl)imidazo[1,2-a]pyrazine

The title compound was made by mesylation of the compound of Example 24 in a manner analogous to that described in Example 14, m.p. 220-222$^{\circ}$C.

Example 5

2-(2-methoxy-4-carbamoylphenyl)-8-methoxy-imidazo[1,2-a]pyrazine hydrochloride

The title compound was prepared from the product of Example 1, in a manner analogous to that described in Example 2, m.p. 195-197$^{\circ}$C.

Example 6

2-(2-methoxy-4-acetylphenyl)-8-chloro-imidazo[1,2-a]pyrazine

i) 3-Methoxy-4-cyanobenzoic acid

3-methoxy-4-aminobenzoic acid (19g) was suspended in a mixture of Conc HCl (12ml) and water (245ml) and stirred at 0-5$^{\circ}$. A solution of sodium nitrite (1 eq.) in water (18ml) was added dropwise to the above suspension maintaining the temperature between 0-5$^{\circ}$. The mixture was stirred at 0-5$^{\circ}$ for 45 min.

The greenish brown solution of the diazonium salt was added in portions to freshly prepared cuprous cyanide solution [Prepared from Copper II sulphate (34.2g) in water (60ml) containing potassium cyanide (18g)] heated at 60$^{\circ}$C. At the end of addition the dark brown solution was refluxed for 2hr.

Insoluble solid was filtered off and the filtrate acidified with conc HCl. Insoluble solid was filtered and redissolved in saturated sodium bicarbonate solution. The solid that remained insoluble was filtered off and the filtrate re-acidified with Conc. HCl. The pale brown solid was filtered and dried (19g) m.p. 237-240$^{\circ}$C.

(ii) 3-methoxy-4-cyanobenzoyl chloride

3-Methoxy-4-cyanobenzoic acid (14g) was suspended in dry toluene (800ml) and thionylchloride (16ml) added and the mixture refluxed for 3hr. Insoluble solid was filtered off and the filtrate evaporated *in vacuo* to give the product as pale brown solid.

(iii) 3-methoxy-4-cyanoacetophenone

Bis-trimethylsilylmalonate (40.35g) was added to dry ether (250ml) and the solution stirred under $N_2$ and cooled to $-60^0$. n-Butyllithium (98.5ml of 1.6M solution in hexane) was added dropwise during 30 min resulting in the formation of a white pecipitate of lithium salt. This was allowed to warm to $0^0$ and then a solution of 3-methoxy-4-cyanobenzoyl chloride obtained above in dry THF (80ml) was added over 10 min. Stirring was continued at $0-5^0$ for 1 hr and at room temperature for 1 hr.

The resulting mixture was acidified with Conc. HCl and extracted with ethyl acetate (200ml). The organic layer was washed with water, dried and evaporated *in vacuo* to give a brown solid (13g). The solid was dissolved in dioxan (100ml) and refluxed for 2.5 hr. The solvent was removed *in vacuo* and the residue dissolved in dichloromethane (200ml) and washed with saturated $NaHCO_3$ solution and water, dried ($MgSO_4$) and evaporated *in vacuo* to give the title product as a brown solid (9.3g) m.p. 110-111$^0$C.

(iv) 2-methoxy-4-acetyl benzoic acid

3-Methoxy-4-cyano acetophenone (6g) was suspended in 2*N* NaOH (60ml) and the mixture refluxed for 40 min. The dark brown solution was filtered to remove traces of insoluble solid. The filtrate was acidified with glacial acetic acid and extracted with chloroform (3x100ml). The organic layer was dried ($MgSO_4$), decolourised and evaporated *in vacuo* to give a cream coloured solid (4.6g) m.p. 127-128$^0$C.

(v) 2-methoxy-4-acetyl benzoylchloride

2-Methoxy-4-acetylbenzoic acid (5.0g, 0.026M) was suspended in toluene (100ml) and thionylchloride (5ml) was added and the mixture was refluxed for 2hr. The

solvent was removed in vacuo and excess thionylchloride was azeotroped with toluene to leave a pale brown solid.

(vi) 2'-Bromo-2-methoxy-4-acetyl acetophenone

Diazomethane generated from diazald (15.5g) in ether was stirred at $0^{0}$. A suspension of the 2-methoxy-4-acetylbenzoylchloride in ether (50ml) was added dropwise to the diazomethane solution. When the addition was complete the resulting mixture was stirred at $0^{0}$ for 30min. Excess diazomethane was carefully distilled off and the remaining solution was evaporated in vacuo to leave a brownish yellow solid.

The diazoketone obtained above was suspended in dry ether (200ml) and a saturated solution of HBr in ether was added dropwise at room temperature until dissolution of most of the solid occurred. The resulting mixture was stirred at room temperature for 30 min. Traces of insoluble solid were filtered off and the filtrate evaporated in vacuo to give a solid which was purified by flash column chromatography to give the required product. m.p. 98-100$^{0}$C.

(vii) 2-(2-methoxy-4-acetylphenyl)-8-chloro imidazo [1,2-a]pyrazine

2'-Bromo-2-methoxy-4-acetylacetophenone (2.71g, 0.01M) and 2-amino-3-chloropyrazine (1.94g, 0.015m) were dissolved in dry DMF (50ml) and the dark brown solution was stirred under nitrogen at 80-90$^{0}$ for 6hr. The solvent was removed in vacuo and the residue suspended in water and the pH adjusted to 7.0. The insoluble solid was filtered off, dried and purified by flash column chromatography to give the title product m.p. 213-215$^{0}$C.

## Example 7

2-(2-methoxy-4-acetylphenyl)imidazo[1,2-a]pyrazine and its hydrochloride

(i) 2-(2-methoxy-4-acetylphenyl)imidazo[1,2-a]pyrazine

2-(2-methoxy-4-acetylphenyl)-8-chloro-imidazo [1,2-a]pyrazine (0.5g) was dissolved in dry DMF (30ml) and triethylamine (1ml) and 5% palladium on carbon catalyst (160mg) added and the mixture was hydrogenated until uptake of 1 mol equivalent of hydrogen (ca. 40ml). The catalyst was filtered off and the filtrate evaporated in vacuo and the residue was purified by flash column

chromatography to give the title compound as a pale cream solid. m. pt. 192-194°C.

(ii) 2-(2-Methoxy-4-acetylphenyl)imidazo[1,2-a]pyrazine hydrochloride

2-(2-methoxy-4-acetylphenyl)imidazo[1,2-a]pyrazine (0.15g) was suspended in methanol and 1.5 eq of 2N HCl solution was added and the mixture stirred at room temperature for 10 mins. The insoluble solid was filtered off and dried. m.p. 225-228°C (Shrinks ≈ 210°).

Example 8

2-(2-Methoxy-4-methylsulphinylphenyl)-7-methoxyimidazo[1,2-a]pyrimidine

A suspension of 2-methoxy-4-methylthiophenacyl bromide (4 mM, 1.17 g) and 2-amino-4-methoxypyrimidine (5 mM, 625 mg) in EtOH (25 ml) was stirred at reflux for 6 hours. The resulting pale yellow solution was then cooled to 0°C, and the hydrobromide salt filtered off. This was suspended in saturated $NaHCO_3$ solution (50 ml), stirred for 30 minutes, filtered and washed with water. Drying at 80°C/10 mm Hg for 2 hours left a pale yellow solid.

To a solution of the above solid in $CHCl_3$ (25ml) at 0°C was added MCPBA (1 eq, 368mg) in $CHCl_3$. After stirring for one hour, the pale yellow solution was washed with sat $NaHCO_3$ solution and dried over $MgSO_4$. Chromatography on $SiO_2$ (25 gm) eluting with $CHCl_3$ and then 2% MeOH-$CHCl_3$ gave the desired product. This was triturated with EtOAc, filtered and dried at 80°C/10 mm Hg to leave a white crystalline solid, m.pt. 234-235°C.

Example 9

3-Amino-2-(2,4-dimethoxyphenyl)imidazo[1,2-a]pyrimidine hydrochloride

2-(2,4-Dimethoxyphenyl)imidazo[1,2-a]pyrimidine was prepared in a manner analogous to that described in Example 8 and was nitrosated with sodium nitrite in acetic acid and the product reduced by catalytic hydrogenation, m.pt. >200°C (decomp).

Examples 10-12

The following compounds were made in a manner analogous to that described in Example 8:-

10) 6-Methoxy-2-(2-methoxy-4-methylsulphinylphenyl)imidazo[1,2-a]pyrimidine,m.pt. 219-220 °C.
11) 2-(2-Methoxy-4-carbamoylphenyl)imidazo[1,2-a]pyrimidine 0.9 HCl, m.pt. 255-257 °C.
12) 6-Hydroxy-2-(2-methoxy-4-methylsulphinylphenyl)imidazo[1,2-a]pyrimidine, HCl, $0.6H_2O$, m.pt 222-226 °C (decomp.).
13) 6-Methoxy-2-(2,4-dimethoxyphenyl)imidazo[1,2-a]pyrimidine,m.pt. 200-201 °C (decomp.).

Example 14

2-(2-Methoxy-4-methylsulphonyloxyphenyl)imidazo[1,2-a]pyrimidine hydrochloride

2-(Methoxy-4-benzyloxyphenyl)imidazo[1,2-a]pyrimidine was prepared in a manner analogous to that described in Example 8 and debenzylated with thioanisole and trifluoroacetic acid followed by mesylation with methanesulphonyl chloride in pyridine, m.pt. 244-246 °C.

Example 15

6-Methylsulphonyloxy-2-(2-methoxy-4-methylsulphinylphenyl)imidazo[1,2-a]pyrimidine hydrochloride

The compound of Example 3 was mesylated in an analogous manner to that described in Example 14 to yield the title product, m.pt. 184-187 °C (decomp.).

Example 16

6-Amino-2-(2,4-dimethoxyphenyl)imidazo[1,2-a]pyrimidine hydrochloride

6-(4-Methoxyphenylazo)-2-(2,4-dimethoxyphenyl)imidazo [1,2-a]pyrimidine was prepared from 2-aminopyrimidine-5-azo-(4-methoxybenzene) in a manner analogous to that described in Example 8. The resulting protected compound was subjected to catalytic hydrogenation to yield the title product, m.pt. 275-278 °C (decomp.).

Example 17-24

The following compounds were prepared in a manner analogous to that used to prepare the compound of Example 3.

17. 8-(2-Hydroxyethoxy)-2-(2-methoxy-4-methylsulphinylphenyl)imidazo [1,2-a]pyrazine, m.pt. 190-192 °C.

18. 8-*n*-Propoxy-2-(2-methoxy-4-methylsulphinylphenyl)imidazo[1,2-a]pyrazine, m.pt. 150-151 °C.

19. 8-Methoxy-2-(2-methoxy-4-cyanophenyl)imidazo[1,2-a]pyrazine, m.pt. 237-240 °C.

20. 8-Methoxy-2-(2-methoxy-4-methylsulphonylphenyl)imidazo[1,2-a]pyrazine (prepared from the starting material 4-methylsulphonyl-2-methoxyphenacyl bromide), m.pt. 234-236 °C.

21. 8-Hydroxy-2-(2-methoxy-4-methylsulphinylphenyl)imidazo[1,2-a]pyrazine, foams above 150 °C.

22. 6-Methoxy-2-(2-methoxy-4-methylsulphinylphenyl)imidazo[1,2-a]pyrazine, m.pt. 164-165 °C.

23. 8-Methylthio-2-(2-methoxy-4-methylsulphinylphenyl)imidazo[1,2-a]pyrazine, m.pt. 160-161 °C.

24. 8-Methoxy-2-(2-methoxy-4-hydroxyphenyl)imidazo[1,2-a]pyrazine, m.pt 275-276 °C.

Example 25

8-Methylamino-2-(2-methoxy-4-methylthiophenyl)imidazo[1,2-a]pyrazine

The title compound was prepared by treatment of the compound of Example 2 (i) with methylamine, m.pt. 141-143 °C.

Example 26

8-Methylamino-2-(2-methoxy-4-methylsulphinyphenyl)imidazo[1,2-a]pyrazine dihydrochloride

The title compound was prepared by oxidation of the compound of Example 25 with MCPBA, 1 (eq), m.pt. 160-161 °C.

Example 27

2-(2-Methoxy-4-methylsulphonyloxyphenyl)imidazo[1,2-a]pyrazine

2-(2-Methoxy-4-benzyloxyphenyl)imidazo(1,2-a]pyrazine was prepared in a manner analogous to that described in Example 1 and subjected to debenzylation in a manner analogous to that described in Example 14 to yield the title product, m.pt. 197-199 °C.

Example 28

8-Methoxy-2-(2-methoxy-4-hydroxyphenyl)imidazo[1,2-a]pyrazine

8-Methoxy-2-(2-methoxy-4-benzyloxyphenyl)imidazo[1,2-a]pyrazine was prepared in a manner analogous to the preparation of the compound of Example 3, followed by catalytic hydrogenation to yield the title product, m.pt 275-276 °C.

Example 29

8-Methoxy-2-(2-methoxy-4-methylsulphonyloxyphenyl)imidazo[1,2-a]pyrazine

The title compound was prepared by mesylation of the compound of Example 28 in a manner analogous to that described in Example 14, m.pt. 220-222 °C.

Example 30

3-Bromo-2-(2-methoxy-4-methylsulphinylphenyl)imidazo[1,2-a]pyrazine

2-(2-Methoxy-4-methylsulphinylphenyl)imidazo[1,2-a]pyrazine was prepared in a manner analogous to that described in Example 1 (iv) and reacted with N-bromosuccinimide to yield the title product, m.pt. 204-205 °C.

Example 31

2-(2-Methoxy-4-carbamoylphenyl)imidazo[1,2-c]pyrimidine

7-Chloro-2-(2-methoxy-4-cyanophenyl)imidazo[1,2-c]pyrimidine was prepared from 4-amino-6-chloropyrimidine and the appropriate phenacyl bromide in a manner analagous to the method described in Example 2 (i). This was catalytically dechlorinated with 5% palladium on charcoal in the presence of dimethylformamide (DMF) and triethylamine. The dechlorinated product was converted to the title compound in a manner analogous to that described in Example 1 (v), m.pt. 264-266 °C.

Example 32

7-Methoxy-2-(2-methoxy-4-methylsulphinylphenyl)imidazo[1,2-c]pyrimidine.

The title compound was prepared in a manner analogous to that described in Example 31 from 4-amino-6-methoxypyrimidine, m.pt. 179-181 °C.

Example 33

2-(2-Methoxy-4-methylsulphonyloxy)imidazo[1,2-c]pyrimidine hydrochloride

a) 2-(4-Hydroxy-2-methoxyphenyl)imidazo[1,2-c]pyrimidine.

7-Chloro-2-(4-benzyloxy-2-methoxyphenyl)imidazo[1,2-c]pyrimidine was prepared in a manner analogous to that described in Example 31 and dechlorinated, also as described in Example 31, this step also bringing about conversion on the benzyloxy substituent to hydroxy, thereby forming the title compound.

b) 2-(2-Methoxy-4-methylsulphonyloxyphenyl)imidazo[1,2-c]pyrimidine hydrochloride

The title compound was prepared from the compound of (a) in a manner analogous to that described in Example 14, m.pt. >235 °C (decomp.).

Example 34

7-Amino-2-(2-methoxy-4-methylsulphinylphenyl)imidazo[1,2-c]pyrimidine

The title compound was prepared in an analogous manner to that described in Example 31, using as starting material, the corresponding diaminopyrimidine, m.pt. 213-215 °C (decomp.).

Example 35

6-Amino-2-(2-methoxy-4-methylsulphinylphenyl)imidazo[1,2-a]pyrimidine

6-(4-Methoxyphenylazo)-2-(2-methoxy-4-methylthiophenyl)imidazo [1,2-a]pyrimidine was prepared in a manner analogous to that described in Example 16. This was oxidised to the corresponding 4-methylsulphinylphenyl compound by the method described in Example 3, followed by deprotection as described in Example 16, to yield the title product m.pt. 219-221 °C.

Example 36

2-(2,4-Dimethoxyphenyl)-3-hydroxyimidazo[1,2-a]pyrimidine and its hydrochloride

a) 2-Aminopyrimidine N-oxide

This was prepared by the oxidation of 2-aminopyrimidine as described by L.W.Deady, Synth,Commun., 509 (1977).

b) 2-(2,4-Dimethoxyphenyl)-3-hydroxyimidazo[1,2-a]pyrimidine

2-Aminopyrimidine N-oxide (4.5g, 40.5 mmol) and 2,4-dimethoxyphenacyl bromide (10.49g, 40.5 mmol) were heated together in dimethylformamide (90 ml) at 80 °C for 2 hours. After overnight standing the DMF was evaporated with the residue taken upin chloroform and washed with aqueous sodium bicarbonate. The organic phase was dried over $Na_2SO_4$ and the solvent evaporated. The residue was chromatographed on silica using chloroform-methanol (10:1). The product solidified and was recrystallised from ethanol-chloroform, giving 2-(2,4-dimethoxyphenyl)-3-hydroxyimidazo[1,2-a]pyrimidine as a yellow solid. δ ($d^6$-DMSO) 8.26 (1H, dd, H-5 or H-7), 8.03 (1H, dd, H-5 or H-7), 7.35 (1H, d, phenyl), 6.98 (1H, dd, H-6), 6.68 (2H, m, phenyl), 3.84 (3H, s, $OCH_3$), 3.78 (3H, s, $OCH_3$); m/e 271 ($M^+$).

c)2-(2,4-Dimethoxyphenyl)-3-hydroxyimidazo[1,2-a]pyrimidine hydrochloride

The hydrochloride was prepared by dissolving the free base in ethanol-chloroform and treating with ethereal HCl. The salt was recrystallised from ethanol-ethyl acetate,

m.pt. 225-228°C (decomp.). $R_f$ 0.21 ($CHCl_3$-MeOH, 10:1). Found: C, 54.19; H, 4.42; N, 13.32; Cl, 11.57. Calcd. for $C_{14}H_{13}N_3O_3$. HCl. $0.2H_2O$: C, 54.00; H, 4.63; N, 13.50; Cl, 11.41.

Example 37

2-(2-Methoxy-4-methylsulphonylaminophenyl)imidazo[1,2-a]pyrimidine hydrochloride

(i) 4-Azido-2-methoxybenzoic acid

A stirred solution of 4-Amino-2-methoxybenzoic acid (16.7g, 0.10 mole) in 200ml of 11% w/v aqueous sulphuric acid was cooled to 0°C and treated over 15min with a cold solution of sodium nitrite (47.7g, 0.69 mole) in 100ml water. The reaction mixture was stirred for 45min at 0-5°C and then a solution of sodium azide (73.0g, 1.12 mole) in 300ml water was added in a steady stream over 15min, the temperature being maintained below 10°C by vigorous stirring and external cooling. After recooling to 0°C and stirring at this temperature for 45min, the suspension was filtered yielding a straw-coloured solid. This was washed with water and recrystallised from ethanol yielding 4-azido-2-methoxybenzoic acid, mpt. 151-152°C. Anal: Found C,49.9 ; H, 3.50 ; N,21.8%. $C_8H_7N_3O_3$ requires C, 49.7 ; H,3.63; N, 21.8%

(ii) 4-Azido-2-methoxybenzoyl chloride

The compound of (i) (6.0g) and thionyl chloride (50ml) were stirred at reflux under dry nitrogen for 30min. Removal of the excess reagent *in vacuo* and then by azeotroping with benzene gave 4-azido-2-methoxybenzoyl chloride as a pale yellow crystalline solid.

(iii) 2-Methoxy-4-azidoacetophenone

Bis-trimethylsilylmalonate (16.3g) was added to dry ether (100ml) and the solution stirred under $N_2$ and cooled to -60°C. n-Butyllithium (42ml of. 1.6M solution in hexane) was added dropwise during 20min resulting in the formation of a white precipitate of lithium salt. This was allowed to warm to 0°C and then a soluion of the compound of (ii) above (6.90g) in dry THF (70ml) was added over 5min. Stirring was continued at 0-5°C for 1hr. Work up as for Example 6 (iii) gave the title product as a yellow solid, m.p. 51-53°C; $M^+$,191 ; Anal: Found C,56.7; H,4.75; N,21.8%. $C_9H_9H_3O_2$ requires C,56.5, H,4.71, N,22.0%.

(iv) 2-Methoxy-4-azidophenacyl bromide

Phenyltrimethylammonium tribromide (4.51g) was added in a single portion to a solution of the compound of (iii) above (1.91g) in THF (50ml) stirred and cooled at 5°C under nitrogen. The reaction mixture was stirred and cooled at 5°C, filtered, the filtrate and washings diluted with water, and then extracted with ether several times. The combined extracts were washed with aqueous saturated $NaHCO_3$ solution, then water, dried over $MgSO_4$ and the solvent removed in vacuo. Trituration of the residual gum in hexane containing some ethanol yielded a solid which could be crystallised from ether-hexane yielding the title compound as a yellow solid mpt. 104-106°C. Anal: Found C,40.2; H,2.94; N,15.4; $C_9H_8BrN_3O_2$ requires C,40.0; H,2.96; N, 15.6%.

(v) 2-(2-Methoxy-4-azidophenyl)imidazo[1,2-a]pyrimidine hydrobromide

The compound of (iv) above (3.24g) and 2-aminopyrimidine (1.14g) in ethanol (100ml) were stirred at reflux under $N_2$ for 5hr. The reaction mixture was cooled to room temperature and the solid that precipitated was filtered off and recrystallised from aqueous ethanol yielding the title compound as a yellow solid mpt. 181°C (decomp).

(vi) 2-(2-Methoxy-4-aminophenyl)imidazo[1,2-a]pyrimidine

A solution of the hydrobromide of (v) above (1.30g) in ethanol (60ml) containing triethylamine (0.76g) and 5% palladium-charcoal catalyst was shakten under 1 atmos. pressure of hydrogen until t.l.c. indicated that all the starting material had been consumed, ca 2hr. The catalyst was removed by

filtration through hyflo and the filtrate evaporated to dryness in vauco. The residual material was purified by column chromatography on silica gel euluting with $CHCl_3$-MeOH (8:1) yielding the title compound as a yellow solid, mpt. 224 °C (decomp.).

(vii) 2-(2-Methoxy-4-methylsulphonylaminophenyl)imidazo[1,2-a]pyrimidine hydrochloride

To a stirred solution of the compound of (vi) above (0.50g,) in dry pyridine (45ml) to 0 °C was added, over 30min, a solution of mesyl chloride (0.23g) in dry pyridine (10ml). The reaction mixture was stirred at 0-5 °C for 17hr, the pyridine removed in vacuo, and the residue triturated with water and chloroform. The resulting solid was filtered off and recrystallised from ethanolic hydrogen chloride to give the title compound as a yellow solid, mpt. 260-263 °C (decomp.). Anal: Found C 47.1; H, 4.20; N,15.7; $C_{14}H_{14}N_4O_3S$. HCl requires C 47.3; H, 4.23; N 15.8%.

Example 38

2-(4-Acetamido-2-methoxyphenyl)-8-methoxyimidazo[1,2-a]pyrazine

(i) 2-(4-Azido-2-methoxyphenyl)-8-chloroimidazo [1,2-a]pyrazine

2-Amino-3-chloropyrazine (3.38g, 26.1 mmol) and 2-methoxy-4-azidophenacyl bromide (4.7g, 17.4 mmol) were dissolved in DMF (90ml) and the solution was heated at 90 °C for 6 hrs. DMF was removed in vacuo and the residue was triturated with ice-water (100ml) and sat. $NaHCO_3$(100ml). The residue was then dissolved in $CHCl_3$ (50ml) and washed with sat. $NaHCO_3$ and dried over $MgSO_4$. Chromatography on silica, eluting with $CHCl_3$, gave the desired product as a pale brown solid, m.pt. 174-182 °C.

(ii) 2-(4-Azido-2-methoxyphenyl)-8-methoxyimidazo[1,2-a]pyrazine

The compound of (i) above (1g, 3.33 mmol) and sodium methoxide (0.36g, 6.66 mmol) were heated under reflux in methanol (40 ml) for 1hr. Methanol was evaporated and the residue was purified by chromatography on silica, eluting with $CHCl_3$, to give the desired product as a pale brown solid, m.pt. 154-158 °C.

(iii) 2-(4-Amino-2-methoxyphenyl)-8-methoxyimidazo[1,2-a]pyrazine

The compound of (ii) above (0.82g, 2.76 mmol) was hydrogenated in ethanol (45ml) in the presence of triethylamine (0.77 ml, 5.52 mmol) and 5% palladium on carbon catalyst (82 mg). The catalyst was filtered off and the filtrate evaporated in vacuo, was purified by chromatography on silica, eluting with $CHCl_3$, to give the desired product as an orange solid, m.pt. 215-226°C.

(iv) 2-(4-Acetamido-2-methoxyphenyl)-8-methoxyimidazo[1,2-a]pyrazine

Acetic anhydride (0.1 ml, 1.1 mmol) was added to a stirred suspension the compound of (iii) above (0.15g, 0.55 mmol) in pyridine (2.5ml) at room temperature. After 30min. methanol was added and after a further period of 15min. $CHCl_3$ was added. The organic solution was washed with sat. $NaHCO_3$ and dried over $MgSO_4$. Chromatography on silica, eluting with 2% MeOH-$CHCl_3$, gave the desired product as a pale yellow solid, m.pt. 231-235°C.

Example 39

2-(2-Methoxy-4-sulphamoylphenyl)-8-methoxyimidazo[1,2-a]pyrazine

(i) 2-(4-Chlorosulphonyl-2-methoxyphenyl)-8-methoxyimidazo[1,2-a]pyrazine

A solution of sodium nitrite (0.20g, 2.86 mmol) in water (0.5ml) was added dropwise to a suspension of 2-(4-amino-2-methoxyphenyl)-8-methoxyimidazo [1,2-a]pyrazine (0.52g, 1.92 mmol) in acetic acid (1ml) and conc. HCl (1ml) at 0-5°C. The reaction was stirred at 5°C for 30 min and then cooled. to -15°C. Cupric chloride (96 mg, , 0.56 mmol) and 6.5M $SO_2$ in acetic acid (1.4ml) were added. The reaction was stirred at 0.5°C for 48hr. Water (5ml) was added and the desired product was filtered off as a yellow solid, m.pt 284°C (decomp. 184°C).

(ii) 2-(2-Methoxy-4-sulphamoylphenyl)-8-methoxyimidazo[1,2-a]pyrazine

The compound of (i) above (0.1g, 0.28 mmol) was suspended in THF (1ml) and cooled in an ice bath. Conc. ammonia (1.5ml) was added dropwise and the mixture was allowed to stand at room temperature overnight. The desired product was filtered off as a cream solid, m.pt. 215-240°C.

Example 40

2-(2-Methoxy-4-N-methylsulphamoylphenyl)-8-methoxyimidazo[1,2-a]pyrazine

The title compound was prepared in an analogous manner to that described in Example 39, m.pt. 218-230°C.

Example 41

2-(4-N,N-dimethylsulphamoyl-2-methoxyphenyl)-8-methoxyimidazo[1,2-a]pyrazine

The title compound was prepared in an analogous manner to that described in Example 39, m.pt. 167-169°C.

Example 42

2-(4-Acetamido-2-methoxyphenyl)imidazo[1,2-a]pyrazine

(i) 2-(4-Amino-2-methoxyphenyl)-imidazo[1,2-a]pyrazine

2-(4-Azido-2-methoxyphenyl)-8-chloroimidazo[1,2-a]pyrazine (0.9g, 3 mmol) was hydrogenated in DMF (50ml) in the presence of triethylamine (3.6 mml, 25.9 mmol) and 10% palladium on carbon catalyst (90mg). The catalyst was filtered off and the filtrate, evaporated in vacuo, was purified by chromatography on silica, eluting with 2% MeOH-$CHCl_3$, to give the desired product as a yellow solid, m.pt. 208-215°C.

(ii) 2-(4-Acetamido-2-methoxyphenyl)-imidazo[1,2-a]pyrazine

Acetic anhydride (0.03ml, 0.33 mmol) was added to a stirred solution of the compound of (i) above (40 mg, 0.17 mmol) in pyridine (1ml). After 3 hr. methanol was added and after a further period of 15 min. $CHCl_3$ was added. The organic solution was washed with sat. $NaHCO_3$ and dried over $MgSO_4$. Chromatography on silica, eluting with 2% MeOH-$CHCl_3$, gave the desired product as a pale yellow solid, m.pt. 240-254°C.

Example 43

2-(2-Methoxy-4-ureidophenyl)imidazo[1,2-a]pyrimidine

2-(2-Methoxy-4-aminophenyl)imidazo[1,2-a]pyrimidine was prepared in an analogous manner to that described in Example 8 and converted to the title product by treatment with potassium cyanate in dilute hydrochloric acid, m.pt 265 °C (decomp.).

Example 44

2-(2-Methoxy-4-methylsulphonylaminophenyl)imidazo[1,2-a]pyrazine

The title compound was prepared in a manner analogous to that described in Example 1, m/e 318 ($M^+$), Rf 0.3 ($CHCl_3$ MeOH, 10:1).

Pharmaceutical Formulations

The following Examples illustrate pharmaceutical formulations according to the present invention wherein the active compound may be any compound of formula (I) defined above, for example, 2-(2-methoxy-4-carbamoylphenyl)imidazo[1,2-a]pyrazine hydrochloride.

Example A

Tablet Formulation

| | |
|---|---|
| Active compound (as base) | 100 mg |
| Lactose | 100 mg |
| Sodium starch glycollate | 20 mg |
| Polyvinylpyrrolidone | 4 mg |
| Magnesium stearate | 2 mg |
| | 226 mg |

Mix the active compound with the lactose and the sodium starch glycollate. Granulate the mixture with a solution of polyvinylpyrrolidone in 50% aqueous alcohol. Dry the granulate and mix in the magnesium stearate. Compress to tablets of average weight 226 mg.

Example B

Capsule Formulation

| | |
|---|---|
| Active compound (as base) | 100 mg |
| Lactose | 100 mg |
| Starch | 30 mg |
| Methylcellulose | 4 mg |
| Stearic Acid | 4 mg |
| | 238 mg |

Mix the active compound with the lactose and the starch. Granulate with a solution of the methylcellulose in water. Dry and mix in the stearic acid. Fill 238 mg into a hard gelatin capsule.

Example C
IV Injection (Freeze Dried)

| | | |
|---|---|---|
| Active compound (as hydrochloride) | | 50 mg |
| Mannitol | | 50 mg |
| Water for Injection | to | 2 ml |

Dissolve the active compound in the Water for Injections. Sterilise the solution by passage through a membrane filter, 0.2 μ pore size, collecting the filtrate in a sterile glass receiver. Fill into sterile glass 2 ml vials under aseptic conditions and secure with aluminium seals.

The injection is reconstituted before administration by the addition of a convenient volume of Water for Injection or sterile saline solution when a large volume infusion is required.

Example D
IV Injection (Multidose vial)

| | |
|---|---|
| Active compound (as hydrochloride) | 250 mg |
| Benzyl Alcohol | 0.075 ml |
| Water for Injection | 5 ml |

Dissolve the benzyl alcohol in Water for Injection. Add and dissolve the active compound. Make up to volume with Water for Injection. Pass through a membrane filter, 0.2 μ pore size, collecting the filtrate in a sterile glass receiver. Fill into sterile glass vials. Close the vials with sterile rubber closures and secure with aluminium seals.

Example E

Suppository

| | | |
|---|---|---|
| Compound (as base) | | 100 mg |
| Suppository Base (Massa Esterinum C) | to | 2 g |

Melt the suppository base at 40°C. Incorporate the active compound in fine powder form in the molten base and mix until homogeneous. Pour the mixture into suitable moulds, 2 g per mould and allow to set.

Biological Activity

Determination of in vivo Inotropic and Vasodilatory Activity

Compounds according to the present invention were tested *in vivo* in comparison with the compound 2-(2-methoxy-4-methylsulphinylphenyl)imidazo[1,2-a]pyrazine (hereinafter referred to as compound 'X' disclosed in UK patent specification No. 2 132 203 A. The compounds were tested in anaesthetised, open-chest beagle dogs by bolus intravenous injection to produce a dose-related positive inotropic stimulation (as measured by the increase in the maximum rate of change of the left ventricular pressure - L.V. dp/dt) over the dose range of 0.003 - 3.0 mg/kg. This was accompanied by a dose-related increase in aortic blood flow, and a fall in systemic blood pressure (see Table below). In these preparations, the duration of inotropic response was estimated by measuring the time in which a 50% increase in L.V. dp/dt would return to pre-dose levels. The duration is shown in the table below and for some of the compounds is given as a range.

## Table

The effective intravenous dose of compound to produce a 50% increase in L.V. dP/dt ($ED_{50}$ L.V. dP/dt), the duration of inotropic response ($T_r$) and a 30% decrease in diastolic blood pressure ($ED_{30}$D.B.P.) in anaesthetised open-chest beagle dogs.

| Compound | $ED_{50}$L.V. dP/dt mg/kg | $ED_{30}$D.B.P. mg/kg | Selectivity $ED_{50}$L.V. dP/dt / $ED_{30}$D.B.P. | $T_r$ mins. |
|---|---|---|---|---|
| X | 0.75 | 0.028 | 26.8 | 5 |
| Example 1 | 0.02 | 0.05 | 0.4 | >30 |
| Example 3 | 0.91 | 1.17 | 0.08 | 60 |
| Example 4 | 0.07 | 0.06 | 1.22 | 45-60 |
| Example 5 | 0.06 | 0.15 | 0.39 | 30-45 |
| Example 7 | 0.03 | 0.10 | 0.30 | 10 |

RMW/OLM/8th May 1985

Claims:-

1. A compound of formula (I)

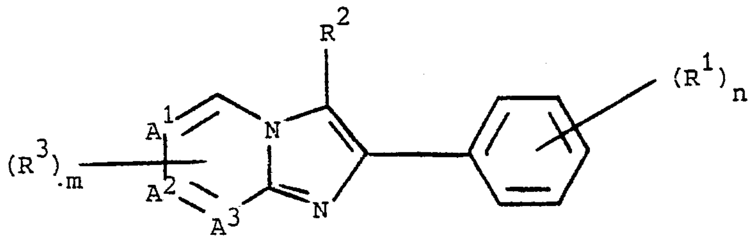

(I)

wherein

n is 1, 2 or 3;

m is 0, 1, 2 or 3;

any one of $A^1$, $A^2$ and $A^3$ is nitrogen and the other two are CH;

$R^1$ groups are independently selected from those specified in (a) and (b) below, namely:-

(a) cyano, hydroxy, groups of formula $-S(O)_xR^a$ in which x is 0, 1 or 2 and $R^a$ is a $C_{1-4}$ alkyl group and groups of formula $-OR^b$ in which $R^b$ is allyl or a $C_{1-4}$ alkyl group, which alkyl group is optionally substituted by one or more radicals selected from halo, $C_{1-4}$ alkoxy and groups of formula $-S(O)_xR^a$ in which x and $R^a$ are as defined above;

(b) $C_{1-4}$ alkanoyl, $C_{1-4}$ alkanoylamino, 2-methyl-1,3-dioxalan-2-yl, sulphamoyl, N-$C_{1-4}$ alkylsulphamoyl, N,N-di-$C_{1-4}$alkylsulphamoyl, $C_{1-4}$ alkylsulphonyloxy, $C_{1-4}$ alkylsulphonylamino, aminosulphonyloxy, N-$C_{1-4}$alkylaminosulphonyloxy, N,N-di-$C_{1-4}$ alkylaminosulphonyloxy, ureido, 3-$C_{1-4}$ alkylureido, 3,3-di-$C_{1-4}$ alkylureido, aminosulphonylamino, ($C_{1-4}$alkylaminosulphonyl)amino,(di-$C_{1-4}$ alkylaminosulphonyl) amino, carboxy, carboxlic derivatives (selected from carbamoyl, N-$C_{1-4}$ alkylcarbamoyl, N,N-di-$C_{1-4}$ alkylcarbamoyl, carboxylic acid halides, $C_{1-4}$

alkoxycarbonyl, hydroxy-$C_{1-4}$alkoxycarbonyl and hydroxymethylene) and groups of formula $-OR^c$ in which $R^c$ is a straight or branched aliphatic moiety having 1 to 4 carbon atoms, optionally substituted by onr or more radicals independently selected from halo, $C_{1-4}$ alkoxy and groups of formula $-S(O)_xR^a$ in which x and $R^a$ are as defined above, provided that $R^c$ is not a group as defined for $R^b$ above;

$R^2$ is selected from hydrogen, halo, amino, hydroxy, $C_{1-4}$ alkyl and $C_{1-4}$ alkoxy;

$R^3$ groups are independently selected from halo, carboxy, amino, $C_{1-4}$alkylamino hydroxy, $C_{1-4}$ alkoxy, hydroxy-$C_{1-4}$alkoxy, $C_{1-4}$ alkylthio and $C_{1-4}$alkylsulphonyloxy;

provided that when $R^2$ is hydrogen and either m is 0 or m is 1 and $R^3$ represents halo, then at least one $R^1$ group must be selected from (b) above; and

when n and m are both 1, and $R^2$ is H, then when $A^1$ is nitrogen, $A^2$ and $A^3$ are both CH and $R^1$ is a 4-methoxy group, $R^3$ is not a 7-methoxy group, and when $A^3$ is nitrogen and $A^1$ and $A^2$ are both CH and $R^1$ is a 4-methylsulphonyl group, $R^3$ is not a 6-methoxy group;

or a physiologically acceptable salt and/or N-oxide thereof.

2. A compound as claimed in claim 1, wherein

n is 2;

m is 0 or 1;

$R^1$ groups are independently selected from cyano, hydroxy, groups of formula $-S(O)_xR^a$ in which and $R^a$ are as defined in formula (I), groups of formula $-OR^b$ in which $R^b$ is a $C_{1-4}$ alkyl group, $C_{1-4}$akanoyl, $C_{1-4}$ alkanoylamino, sulphamoyl, N-$C_{1-4}$ alkylsulphamoyl, N,N,-di-$C_{1-4}$alkylsulphamoyl, $C_{1-4}$ alkylsulphonyloxy, $C_{1-4}$ alkylsulphonylamino, ureido, carbamoyl and aminosulphonylamino; and

$R^2$ is selected from hydrogen, halo, amino and hydroxy;

$R^3$ (where present) is selected from halo, amino, $C_{1-4}$alkylamino, hydroxy, $C_{1-4}$alkoxy, hydroxy-$C_{1-4}$alkoxy, $C_{1-4}$alkythio and $C_{1-4}$alkylsulphonyloxy;

or a physiologically acceptable salt thereof.

RMW/OLM/11th June, 1985

3. A compound as claimed in claim 1, wherein $R^1$ groups are independently selected from:-

cyano, hydroxy, groups of formula $-S(O)_xR^a$ in which x is 0, 1 or 2 and $R^a$ is a $C_{1-4}$ alkyl group, sulphamoyl, N-$C_{1-4}$ alkylsulphamoyl, N,N-di-$C_{1-4}$alkylsulphamoyl, $C_{1-4}$ alkylsulphonyloxy, $C_{1-4}$ alkylsulphonylamino, aminosulphonyloxy, N-$C_{1-4}$ alkylaminosulphonyloxy,N-N-di-$C_{1-4}$ alkylaminosulphonyloxy, carboxy, carboxylic acid derivatives (selected from carbamoyl, N-$C_{1-4}$alkylcarbamoyl, N,N-di-$C_{1-4}$ alkylcarbamoyl, carboxylic acid halides, $C_{1-4}$ alkoxycarbonyl, hydroxy-alkoxycarbonyl and hydroxymethylene) and groups of formula $-OR^d$ in which $R^d$ is a straight or branched aliphatic moiety having 1 to 4 carbon atoms, optionally substituted by one or more radicals independently selected from halo, $C_{1-4}$ alkoxy and groups of formula $-S(O)_xR^a$ in which x and $R^a$ are as defined above;

$R^2$ is selected from hydrogen, hydroxy, $C_{1-4}$ alkyl and $C_{1-4}$ alkoxy;

$R^3$ groups are independently selected from halo, carboxy, amino, hydroxy, $C_{1-4}$ alkoxy, $C_{1-4}$ alkylthio and $C_{1-4}$alkylsulphonyloxy; with the further provisos that:-

when $A^2$ is nitrogen and $A^1$ and $A^3$ are both CH, then $R^3$ is other than amino or $C_{1-4}$ alkylsulphonyloxy, and when $A^2$ is nitrogen and $A^1$ and $A^3$ are both CH, or $A^3$ is nitrogen and $A^1$ and $A^2$ are both CH, then $R^3$ is other than hydroxy;

or a physiologically acceptable salt and/or N-oxide thereof.

4. A compound as claimed in claim 1 or claim 3, wherein $A^2$ is nitrogen and $A^1$ and $A^3$ are both CH, or a physiologically acceptable salt and/or N-oxide thereof.

5. A compound as claimed in any of claims 1, 3 and 4 selected from

8-methoxy-2-(2-methoxy-4-methylsulphinylphenyl) imidazo [1,2-a]pyrazine
2-(4-methylsulphonyloxy-2-methoxyphenyl)-8-methoxy-imidazo[1,2-a]pyrazine
2-(4-carbamoyl-2-methoxyphenyl)-8-methoxy-imidazo[1,2-a]pyrazine

and physiologically acceptable salts and/or N-oxides thereof.

6. 2-(2-Methoxy-4-carbamoylphenyl)-imidazo[1,2-a]pyrazine and its N-oxides and physiologically acceptable salts.

RMW/OLM/11th June, 1985

7. A pharmaceutical formulation comprising a compound of formula (I)

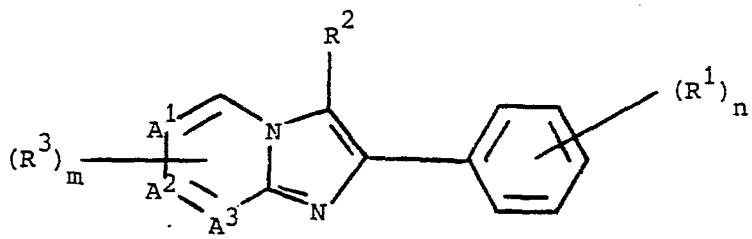

(I)

wherein

n is 1, 2 or 3;

m is 0, 1, 2 or 3;

any one of $A^1$, $A^2$ and $A^3$ is nitrogen and the other two are CH;

$R^1$ groups are independently selected from those specified in (a) and (b) below, namely:-

(a) cyano, hydroxy, groups of formula $-S(O)_xR^a$ in which x is 0, 1 or 2 and $R^a$ is a $C_{1-4}$ alkyl group and groups of formula $-OR^b$ in which $R^b$ is allyl or a $C_{1-4}$ alkyl group, which alkyl group is optionally substituted by one or more radicals selected from halo, $C_{1-4}$ alkoxy and groups of formula $-S(O)_xR^a$ in which x and $R^a$ are as defined above;

(b) $C_{1-4}$ alkanoyl, $C_{1-4}$ alkanoylamino, 2-methyl-1,3-dioxalan-2-yl, sulphamoyl, N-$C_{1-4}$ alkylsulphamoyl, N,N-di-$C_{1-4}$alkylsulphamoyl, $C_{1-4}$ alkylsulphonyloxy, $C_{1-4}$ alkylsulphonylamino, aminosulphonyloxy, N-$C_{1-4}$alkylaminosulphonyloxy, N,N-di-$C_{1-4}$ alkylaminosulphonyloxy, ureido, 3-$C_{1-4}$ alkylureido, 3,3-di-$C_{1-4}$ alkylureido, aminosulphonylamino, ($C_{1-4}$alkylaminosulphonyl)amino,(di-$C_{1-4}$ alkylaminosulphonyl) amino, carboxy, carboxlic derivatives (selected from carbamoyl, N-$C_{1-4}$ alkylcarbamoyl, N,N-di-$C_{1-4}$ alkylcarbamoyl, carboxylic acid halides, $C_{1-4}$ alkoxycarbonyl, hydroxy-$C_{1-4}$alkoxycarbonyl and hydroxymethylene) and groups of formula $-OR^c$ in which $R^c$ is a straight or branched aliphatic moiety having 1 to 4 carbon atoms, optionally substituted by onr or more radicals independently selected from halo, $C_{1-4}$ alkoxy and groups of formula $-S(O)_xR^a$ in which x and $R^a$ are as defined above, provided that $R^c$ is not a group as defined for $R^b$ above;

$R^2$ is selected from hydrogen, halo, amino, hydroxy, $C_{1-4}$ alkyl and $C_{1-4}$ alkoxy;

$R^3$ groups are independently selected from halo, carboxy, amino, $C_{1-4}$alkylamino hydroxy, $C_{1-4}$ alkoxy, hydroxy-$C_{1-4}$alkoxy, $C_{1-4}$ alkylthio and $C_{1-4}$alkylsulphonyloxy;

provided that when $R^2$ is hydrogen and either m is 0 or m is 1 and $R^3$ represents halo, then at least one $R^1$ group must be selected from (b) above;

or a physiologically acceptable salt and/or N-oxide thereof; and at least one pharmaceutical carrier or excipient.

8. Use of a compound of formula (I) as defined in claim 7 or a physiologically acceptable salt and/or N-oxide thereof, for use in the manufacture of inotropic agents.

9. Use according to claim 8, wherein the inotropic agent is for the treatment or prevention of congestive heart failure or of myocardial insufficiency.

10. A process for the preparation of a compound of formula (I) as defined in claim 1 or a physiologically acceptable salt and/or N-oxide thereof, the method comprising:-

a) reacting a compound of formula

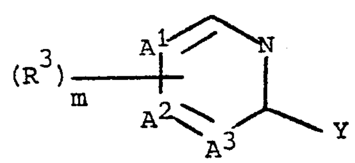

(II)

(wherein $A^1$, $A^2$, $A^3$, m and $R^3$ are as defined above and Y is an amino group or a displaceable radical, e.g. hydrogen or a halogen atom such as chlorine) with a compound of formula

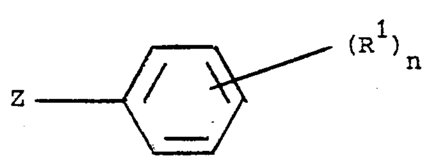

(III)

(wherein $R^1$ and n are as defined above or precursor groups therefor and Z represents a group capable of reacting with group Y to form an imidazo ring system with consequential formation of a compound of formula (I) or an acid addition salt thereof);

b) reacting a compound of formula

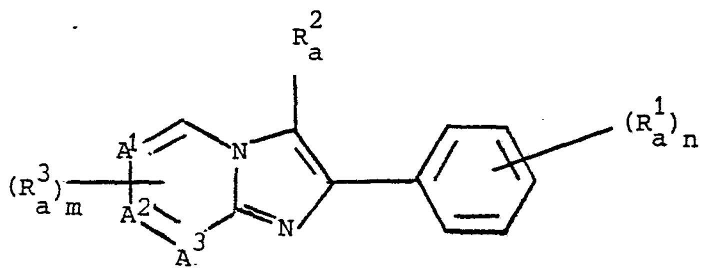

(IV)

(wherein $A^1$, $A^2$, $A^3$, n and m are as defined above and each of $R^1_a$ to $R^3_a$ independently represents a group as defined respectively for $R^1$ to $R^3$ above or a precursor group therefor, provided that at least one of $R^1_a$ to $R^3_a$ represents such a precursor group) or an acid addition salt thereof with an agent serving to effect conversion of the said precursor group(s) into the desired group(s);

c) radical arylation of a compound of formula

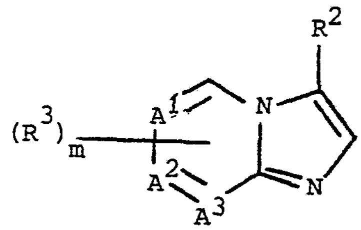

(V)

(wherein $A^1$, $A^2$, $A^3$, $R^2$, $R^3$, and m are as hereinbefore defined) by reaction with a compound of formula

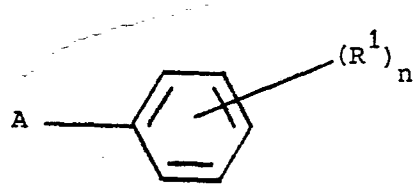

(VI)

(wherein $R^1$ and n are as hereinbefore defined and A represents a displaceable radical, e.g. a diazonium halide, for example the chloride); or

(d) reacting a compound of formula

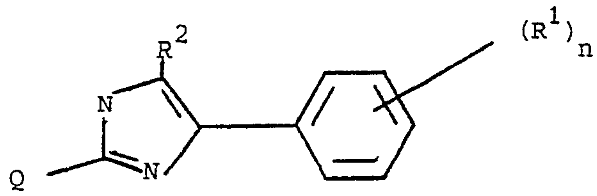

(VII)

(wherein $R^1$, $R^2$ and n are as defined above) with a reagent such that the reagent and Q (in formula (VII)) are chosen so as to react to complete formation of the ring

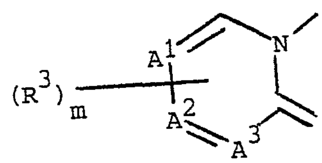

(wherein $A^1$, $A^2$, $A^3$, $R^3$ and m are as defined above);

and when a compound of formula (I) is formed, optionally converting the said compound into the N-oxide and/or acid addition salt thereof.

Claims:-

1. A process for the preparation of a compound of formula (I)

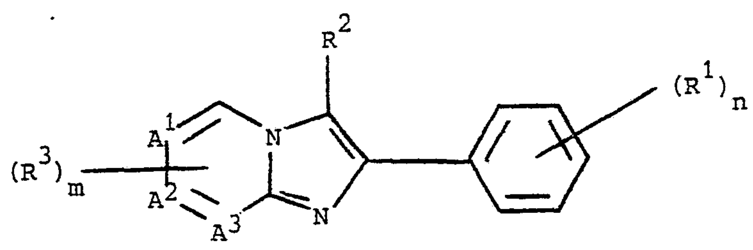

(I)

wherein

n is 1, 2 or 3;

m is 0, 1, 2 or 3;

any one of $A^1$, $A^2$ and $A^3$ is nitrogen and the other two are CH;

$R^1$ groups are independently selected from those specified in (a) and (b) below, namely:-

(a) cyano, hydroxy, groups of formula $-S(O)_xR^a$ in which x is 0, 1 or 2 and $R^a$ is a $C_{1-4}$ alkyl group and groups of formula $-OR^b$ in which $R^b$ is allyl or a $C_{1-4}$ alkyl group, which alkyl group is optionally substituted by one or more radicals selected from halo, $C_{1-4}$ alkoxy and groups of formula $-S(O)_xR^a$ in which x and $R^a$ are as defined above;

(b) $C_{1-4}$ alkanoyl, $C_{1-4}$ alkanoylamino, 2-methyl-1,3-dioxalan-2-yl, sulphamoyl, N-$C_{1-4}$ alkylsulphamoyl, N,N-di-$C_{1-4}$alkylsulphamoyl, $C_{1-4}$ alkylsulphonyloxy, $C_{1-4}$ alkylsulphonylamino, aminosulphonyloxy, N-$C_{1-4}$alkylaminosulphonyloxy, N,N-di-$C_{1-4}$ alkylaminosulphonyloxy, ureido, 3-$C_{1-4}$ alkylureido, 3,3-di-$C_{1-4}$ alkylureido, aminosulphonylamino, ($C_{1-4}$alkylaminosulphonyl)amino,(di-$C_{1-4}$ alkylaminosulphonyl) amino, carboxy, carboxlic derivatives (selected from carbamoyl, N-$C_{1-4}$ alkylcarbamoyl, N,N-di-$C_{1-4}$ alkylcarbamoyl, carboxylic acid halides, $C_{1-4}$ alkoxycarbonyl, hydroxy-$C_{1-4}$alkoxycarbonyl and hydroxymethylene) and groups of formula $-OR^c$ in which $R^c$ is a straight or branched aliphatic moiety having 1 to 4

carbon atoms, optionally substituted by onr or more radicals independently selected from halo, $C_{1-4}$ alkoxy and groups of formula $-S(O)_xR^a$ in which x and $R^a$ are as defined above, provided that $R^c$ is not a group as defined for $R^b$ above;

$R^2$ is selected from hydrogen, halo, amino, hydroxy, $C_{1-4}$ alkyl and $C_{1-4}$ alkoxy;

$R^3$ groups are independently selected from halo, carboxy, amino, $C_{1-4}$alkylamino hydroxy, $C_{1-4}$ alkoxy, hydroxy-$C_{1-4}$alkoxy, $C_{1-4}$ alkylthio and $C_{1-4}$alkylsulphonyloxy;

provided that when $R^2$ is hydrogen and either m is 0 or m is 1 and $R^3$ represents halo, then at least one $R^1$ group must be selected from (b) above; and

when n and m are both 1, and $R^2$ is H, then when $A^1$ is nitrogen, $A^2$ and $A^3$ are both CH and $R^1$ is a 4-methoxy group, $R^3$ is not a 7-methoxy group, and when $A^3$ is nitrogen and $A^1$ and $A^2$ are both CH and $R^1$ is a 4-methylsulphonyl group, $R^3$ is not a 6-methoxy group;

or a physiologically acceptable salt and/or N-oxide thereof, the process comprising:-

a) reacting a compound of formula

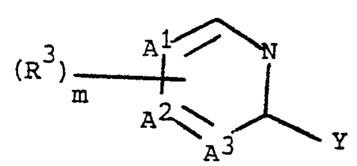

(II)

(wherein $A^1$, $A^2$, $A^3$, m and $R^3$ are as defined above and Y is an amino group or a displaceable radical, e.g. hydrogen or a halogen atom such as chlorine) with a compound of formula

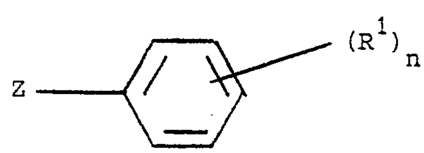

(III)

(wherein $R^1$ and n are as defined above or precursor groups therefor and Z represents a group capable of reacting with group Y to form an imidazo ring system with

consequential formation of a compound of formula (I) or an acid addition salt thereof);

b) reacting a compound of formula

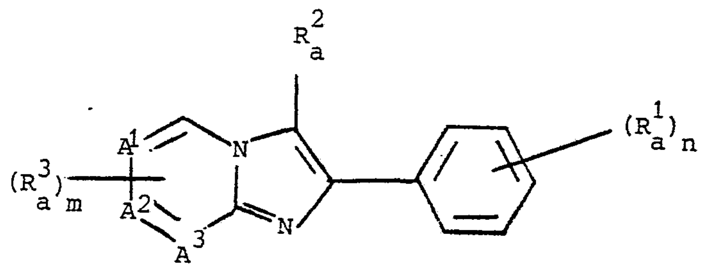

(IV)

(wherein $A^1$, $A^2$, $A^3$, n and m are as defined above and each of $R_a^1$ to $R_a^3$ independently represents a group as defined respectively for $R^1$ to $R^3$ above or a precursor group therefor, provided that at least one of $R_a^1$ to $R_a^3$ represents such a precursor group) or an acid addition salt thereof with an agent serving to effect conversion of the said precursor group(s) into the desired group(s);

c) radical arylation of a compound of formula

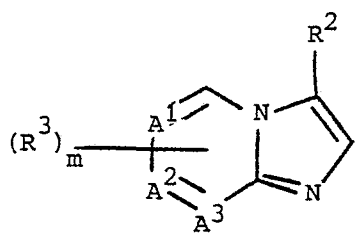

(V)

(wherein $A^1$, $A^2$, $A^3$, $R^2$, $R^3$, and m are as hereinbefore defined) by reaction with a compound of formula

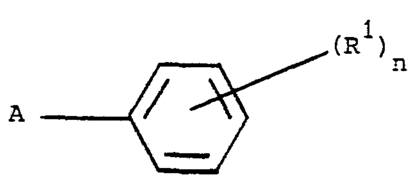

(VI)

(wherein $R^1$ and n are as hereinbefore defined and A represents a displaceable radical, e.g. a diazonium halide, for example the chloride); or

(d) reacting a compound of formula

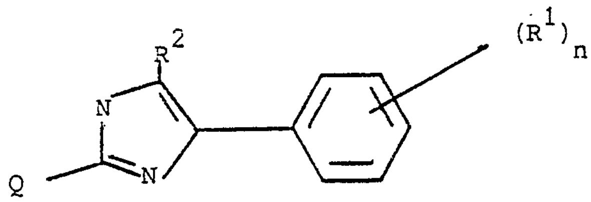

(VII)

(wherein $R^1$, $R^2$ and n are as defined above) with a reagent such that the reagent and Q (in formula (VII)) are chosen so as to react to complete formation of the ring

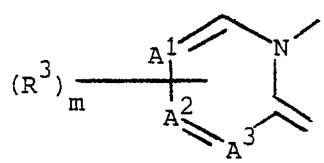

(wherein $A^1$, $A^2$, $A^3$, $R^3$ and m are as defined above);

and when a compound of formula (I) is formed, optionally converting the said compound into the N-oxide and/or acid addition salt thereof.

2. A process as claimed in claim 1, wherein the compound so prepared is a compound wherein:-

n is 2;

m is 0 or 1;

$R^1$ groups are independently selected from cyano, hydroxy, groups of formula -$S(O)_xR^a$ in which and $R^a$ are as defined in formula (I), groups of formula -$OR^b$ in which $R^b$ is a $C_{1-4}$ alkyl group, $C_{1-4}$akanoyl, $C_{1-4}$ alkanoylamino, sulphamoyl, N-$C_{1-4}$ alkylsulphamoyl, N,N,-di-$C_{1-4}$alkylsulphamoyl, $C_{1-4}$ alkylsulphonyloxy, $C_{1-4}$alkylsulphonylamino, ureido, carbamoyl and aminosulphonylamino; and

$R^2$ is selected from hydrogen, halo, amino and hydroxy;

$R^3$ (where present) is selected from halo, amino, $C_{1-4}$alkylamino,

hydroxy, $C_{1-4}$alkoxy, hydroxy-$C_{1-4}$alkoxy, $C_{1-4}$alkythio and $C_{1-4}$alkylsulphonyloxy.

or a physiologically acceptable salt thereof.

3. A process as claimed in claim 1, whérein the compound so prepared is a compound wherein $R^1$ groups are independently selected from:-

cyano, hydroxy, groups of formula $-S(O)_xR^a$ in which x is 0, 1 or 2 and $R^a$ is a $C_{1-4}$ alkyl group, sulphamoyl, N-$C_{1-4}$ alkylsulphamoyl, N,N-di-$C_{1-4}$alkylsulphamoyl, $C_{1-4}$ alkylsulphonyloxy, $C_{1-4}$ alkylsulphonylamino, aminosulphonyloxy, N-$C_{1-4}$ alkylaminosulphonyloxy,N-N-di-$C_{1-4}$ alkylaminosulphonyloxy, carboxy, carboxylic acid derivatives (selected from carbamoyl, N-$C_{1-4}$alkylcarbamoyl, N,N-di-$C_{1-4}$ alkylcarbamoyl, carboxylic acid halides, $C_{1-4}$ alkoxycarbonyl, hydroxy-alkoxycarbonyl and hydroxymethylene) and groups of formula $-OR^d$ in which $R^d$ is a straight or branched aliphatic moiety having 1 to 4 carbon atoms, optionally substituted by one or more radicals independently selected from halo, $C_{1-4}$ alkoxy and groups of formula $-S(O)_xR^a$ in which x and $R^a$ are as defined above;

$R^2$ is selected from hydrogen, hydroxy, $C_{1-4}$ alkyl and $C_{1-4}$ alkoxy;

$R^3$ groups are independently selected from halo, carboxy, amino, hydroxy, $C_{1-4}$ alkoxy, $C_{1-4}$ alkylthio and $C_{1-4}$alkylsulphonyloxy; with the further provisos that:-

when $A^2$ is nitrogen and $A^1$ and $A^3$ are both CH, then $R^3$ is other than amino or $C_{1-4}$ alkylsulphonyloxy, and when $A^2$ is nitrogen and $A^1$ and $A^3$ are both CH, or $A^3$ is nitrogen and $A^1$ and $A^2$ are both CH, then $R^3$ is other than hydroxy;

or a physiologically acceptable salt and/or N-oxide thereof.

4. A process as claimed in claim 1 or claim 3, wherein is the compound of formula (I) or physiologically acceptable salt and/or N-oxide thereof, $A^2$ is nitrogen and $A^1$ and $A^3$ are both CH.

5. A process as claimed in any of claims 1,3 and 4, wherein the compound so prepared is selected from

8-methoxy-2-(2-methoxy-4-methylsulphinylphenyl) imidazo [1,2-a]pyrazine
2-(4-methylsulphonyloxy-2-methoxyphenyl)-8-methoxy-imidazo[1,2-a]pyrazine
2-(4-carbamoyl-2-methoxyphenyl)-8-methoxy-imidazo[1,2-a]pyrazine

RMW/OLM/11th June 1985

and physiologically acceptable salts and/or N-oxide thereof.

6. A process as claimed in any of claims 1,3 and 4, wherein the compound so prepared is selected from 2-(2-Methoxy-4-carbamoylphenyl)-imidazo[1,2-a]pyrazine and its N-oxides and physiologically acceptable salts.

7. A process as claimed in claim 1 (b) or claim 7, comprising acid hydrolysis of 2-(2-methoxy-4-cyanophenyl)imidazo[1,2-a]pyrazine, and optionally converting the resulting product to a physiologically acceptale salt and/or N-oxide thereof.

8. A process as claimed in claimed 1(d) wherein in the compound of Formula (I), $A^2$ is nitrogen and $A^1$ $A^3$ are both CH, the substituent Q in the compound of formula (VII) is formyl and the reagent has the formula

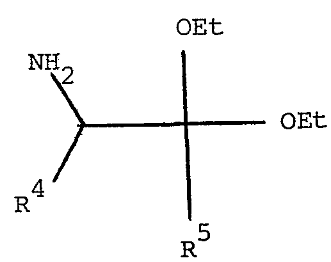

(XI)

(wherein $R^4$ and $R^5$ are independently selected from hydrogen and groups as defined for $R^3$ above).

9. A process for the preparation of a pharmaceutical formulation by:-

a) preparing a compound of formula (I) or a physiologically acceptable salt and/or N-oxide thereof by a process as claimed in any of claims 1-8; and

(b) admixing the resulting product and at least one pharmaceutical carrier or excipient.

RMW/OLM/11th June 1985